# EUROPEAN PATENT SPECIFICATION

(11) **EP 3 684 370 B1**
(45) Date of publication and mention of the grant of the patent: **14.05.2025**
(21) Application number: 19756827.2
(22) Date of filing: 26.02.2019
(51) Int. Cl.: A61K 31/573, A61P 27/02, A61K 31/58, A61K 39/395, A61K 9/00, A61K 45/06

(54) **COMPOSITION AND TREATMENT OF GEOGRAPHIC ATROPHY WITH FLUDROCORTISONE**
ZUSAMMENSETZUNG UND BEHANDLUNG VON GEOGRAPHISCHE ATROPHIE MIT FLUDROCORTISON
COMPOSITION ET TRAITEMENT DE L'ATROPHIE GÉOGRAPHIQUE AVEC DE LA FLUDROCORTISONE

(30) Priority: 26.02.2018 AU 2018900607
(43) Date of publication of application: 29.07.2020
(73) Proprietor: Eye Co Pty Ltd., Balwyn North, Victoria 3104 (AU)
(72) Inventor: PENFOLD, Philip, Leslie, Balwyn North, Victoria 3104 (AU)
(74) Representative: Pföstl, Andreas
(86) International application number: PCT/AU2019/000023
(87) International publication number: WO 2019/161434

(56) References cited:
- WO-A1-2015/089559
- WO-A2-2005/099715
- EVANS JAMES B. ET AL: "New hope for dry AMD?", NATURE REVIEWS DRUG DISCOVERY, vol. 12, no. 7, 1 July 2013 (2013-07-01), GB, pages 501 - 502, XP055807671, ISSN: 1474-1776, Retrieved from the Internet <URL:https://www.nature.com/articles/nrd4038.pdf> DOI: 10.1038/nrd4038
- BANDELLO FRANCESCO ET AL: "Recent advances in the management of dry age-related macular degeneration: A review", F1000RESEARCH, vol. 6, 1 January 2017 (2017-01-01), pages 245, XP055807791, Retrieved from the Internet <URL:https://www.ncbi.nlm.nih.gov/pmc/articles/PMC5428517/pdf/f1000research-6-11493.pdf> DOI: 10.12688/f1000research.10664.1
- WANG Y ET AL: "The role of anti-inflammatory agents in age-related macular degeneration (AMD) treatment", EYE, vol. 25, no. 2, 1 February 2011 (2011-02-01), GB, pages 127 - 139, XP055807873, ISSN: 0950-222X, Retrieved from the Internet <URL:https://www.ncbi.nlm.nih.gov/pmc/articles/PMC3044916/pdf/eye2010196a.pdf> DOI: 10.1038/eye.2010.196
- VAN LOOKEREN CAMPAGNE , MENNO ET AL.: "Mechanisms of age-related macular degeneration and therapeutic opportunities", JOURNAL OF PATHOLOGY, vol. 232, 2014, pages 151 - 164, XP055135931, Retrieved from the Internet <URL:https://onlinelibrary.wiley.com/doi/full/10.1002/path.4266> [retrieved on 20190328], doi:10.1002/path.4266

## Description

### FIELD OF THE INVENTION

The present invention relates to a pharmaceutical composition for use in the treatment of geographic atrophy.

### BACKGROUND TO THE INVENTION

It is widely accepted that there is no medical or surgical treatment for dry AMD.

Aruna Gorusupudi, Kelly Nelson, and Paul S Bernstein noted that a wide variety of nutrients, such as minerals, vitamins, v-3 (n-3) fatty acids, and various carotenoids, have been associated with reducing the risk of AMD. These authors noted that results from the Age-Related Eye Disease Study (AREDS) indicated that supplementation with antioxidants (b-carotene and vitamins C and E) and zinc was associated with a reduced risk of AMD progression and that the AREDS2 follow-up study, was designed to improve upon the earlier formulation, tested the addition of lutein, zeaxanthin, and v-3 fatty acids.

However, Singh states that AREDS failed to show that vitamin supplementation decreased progression to geographic atrophy (GA). GA is an advanced form of AMD that can result in the progressive and irreversible atrophy of retina (photoreceptors, retinal pigment epithelium (RPE) and choriocappillaris). The pathogenesis of GA is multifactorial and is thought to be triggered by intrinsic and extrinsic stressors of the poorly regenerative RPE. The regions of atrophy can look like a map, this explains the term "geographic". The term GA is used interchangeably with the term "dry AMD". Even in AREDS2, when beta-carotene was replaced with lutein/zeaxanthin to decrease the risk of lung cancer, the new formulation also failed to show decreased progression to GA. Singh refers to clinical studies underway at Cole Eye Institute, Cleveland Clinic, to further elucidate and understand the mechanisms of dry AMD and to evaluate new therapeutics directed at slowing the progression. There are two large phase 3 trials underway for the treatment of GA. The FILLY study assesses the safety, tolerability and evidence of activity of multiple intravitreal (IVT) injections of APL-2 (Apellis Pharmaceuticals) for patients with GA. The second is a multicenter, randomized, double-masked, sham-controlled study to investigate IVT injections of lampalizumab in patients with GA.

Singh also refers to another area of research that has sprung from the discovery of complement by-products in drusen which led to associations between complement dysregulation and AMD. Several researchers are now evaluating the complement cascade as a clinical therapeutic target for non-neovascular AMD. Factor D is considered an early component of the alternative pathway that involves complement factor H. Anti-inflammatory agents under development include lampalizumab, fluocinolone, glatiramer acetate, sirolimus, eculizumab and ARC-1905.

Exposure to 24 hours of Bright Continuous Light (BCL) has been shown to induce differential expression of a suite of complement system genes, including classic and lectin components, regulators, and receptors. C1gr1, MCP, Dafl, and C1qTNF6 all modulated in concert with photoreceptor death and AP-1 expression, which reached a peak at 24 hours exposure. C1s and C4a reached peak expression at 3 days after exposure, while expression of C3, C3arl, and C5r1 were maximum at 7 days after exposure. C3 mRNA was detected in ED1- and IBA1-positive microglia/macrophages, in the retinal vessels and optic nerve head and in the subretinal space, particularly at the margins of the emerging lesion. This study indicated that BCL induces the prolonged expression of a range of complement genes. A pathogenic role of complement in age-related macular degeneration (AMD) has been uncovered through gene association studies. These identify a significant association between the Y402H sequence variant in the regulatory gene complement factor H (CFH) with incidence of AMD,^{14 -17} along with other susceptibility variants in complement pathway genes such as C2,18,19 CFB,18,19 and the central component C3.²⁰⁻²⁴. These findings were said to establish complement activation and inflammation as factors that influence the onset and progression of AMD (Rutar *et al*.).

IL-1β is produced by retinal microglia and macrophages and promotes chemokine expression by Müller cells and RPE in retinal degeneration. Targeting IL-1β has been suggested in broadly suppressing chemokine-mediated inflammation in retinal dystrophies such as AMD (Natoli et al).

C3, produced locally by retinal microglia/macrophages, has been implicated as contributing causally to retinal degeneration. Consequently, it has been suggested that C3-targeted gene therapy may prove valuable in slowing the progression of AMD (Natoli et al. b).

Singh also notes that visual cycle inhibitors are in latter-stage development and include fenretinide, ACU-4429 and ALK-001. These compounds down-regulate the visual cycle to decrease the accumulation of the toxic waste products of retinal metabolism. Amyloid-beta has been found in drusen, and RN6G and GSK933776 are in development to regulate amyloid-beta accumulation.

Another class of therapeutics noted by Singh are neuroprotective drugs, which are under development, including UF-021, ciliary neurotrophic factor and brimonidine tartrate intravitreal implant. Topical agents such as MC-1101 are attempting to slow AMD by increasing choroidal perfusion. Stem cell therapies including HuCNS-SC and MA09-hRPE are also under investigation as potential treatments for GA.

EP 0819003 to John and Karen Repine describes a method for treating or preventing the onset or progression of macular degeneration, comprising periodic administration of a glutathione (GSH) enhancing agent alone or in conjunction with at least one of an anti-oxidant or an anti-inflammatory therapy, and possibly in addition to one or more of the symptomatic treatments mentioned above.

PCT/EP2014/056222 to Isarna Therapeutics Gmbh describes an oligonucleotide consisting of 10 to 20 nucleotides of selected regions of the TGF-beta1, TGF-beta2 or TGF-beta3 nucleic acid sequence, which comprises modified nucleotides such as LNA, ENA, polyalkylene oxide-, 2'-fluoro, 2'-O-methoxy and/or 2'-O-methyl modified nucleotides. Compositions or the oligonucleotides were used in a method for the prevention and/or treatment of glaucoma, posterior capsular opacification, dry eye, Marfan or Loeys-Dietz syndrome, riboblastoma, choroidcarcinoma, macular degeneration, such as age-related maculardegeneration, diabetic macular endma, or cataract.

PCT/US2017/014458 to Vitrisa Therapeutics, Inc. discloses methods and compositions involving the use of aptamers for inhibiting complement Factor D and for the treatment of dry age-related macular degeneration, geographic atrophy, wet age-related macular degeneration or Stargardt disease.

JP2015083609 to GENENTECH INC. describes complement inhibitors including those inhibiting the alternative complement pathway, such as Factor D, properdin, Factor B, Factor Ba, and Factor Bb, and the classical complement pathway, such as C3a, C5, C5a, C5b, C6, C7, C8, C9 and C5b-9 for the treatment of ocular related conditions or diseases, such as age-related macular degeneration (AMD), diabetic retinopathy, ocular angiogenesis (such as ocular neovascularization affecting choroidal, corneal, or retinal tissue), and other ocular conditions involving complement activation.

PCT/US2015/020001 to the UNIVERSITY OF FLORIDA RESEARCH FOUNDATION, INC describes methods and compositions for preventing, treating, and/or ameliorating one or more symptoms of inflammation including AMD, arthritis, Bechet's disease, Best macular dystrophy, corneal inflammation, diabetic retinopathy, drusen formation, dry AMD, dry eye, geographic atrophy, glaucomaocular neovascularization, Lupus erythematosus, macular degeneration, Mallatia Leventinese and Doyne honeycomb retinal dystrophy, nephritis, ocular hypertension, ocular inflammation, recurrent uveitis, Sorsby fundus dystrophy, vasculitis, vitreoretinopathy, wet AMD, or related disorders. The pharmaceutical composition comprises a recombinant viral vector that delivers a secretable and cell-penetrating M013 protein or peptide.

PCT/EL2015/051174 to Dvashi Zeev and Pollack Ayala describes compositions for use in treatment of Alzheimer's Disease, and/or an ocular and retinal degenerative disease, such as AMD. The described compositions include effective amounts of H-Leu-Leu-OMe Hydrochloride or Hydrobromide, or a functional derivative thereof.

The reference to any prior art in this specification is not, and should not be taken as, an acknowledgement or any form of suggestion that the prior art forms part of the common general knowledge.

### SUMMARY OF THE INVENTION

The present invention relates to a composition as defined in the claims for use in the treatment of geographic atrophy.

Disclosed is the use of one or more anti-inflammatory in the treatment of dry AMD. The one or more anti-inflammatory comprises one or more glucocorticoid and/or mineralocorticoid or a therapeutically active analogue, derivative, homolog, pharmaceutically acceptable salt or conjugate thereof.

Disclosed is a method of treating dry AMD in a subject, the method comprising administering to the subject a therapeutically effective amount of an anti-inflammatory to thereby treat the dry AMD. The therapeutically effective amount of an anti-inflammatory may be comprised in a pharmaceutical composition.

Disclosed is a pharmaceutical composition comprising a therapeutically effective amount of one or more anti-inflammatory and a pharmaceutically acceptable carrier, diluent or excipient when used to treat dry AMD.

Disclosed is the use of a pharmaceutical composition comprising one or more anti-inflammatory for the manufacture of a medicament for the treatment of dry AMD.

The one or more anti-inflammatory comprises one or more of a COX inhibitor, one or more mineralocorticoid or a therapeutically active analogue, derivative, homolog, pharmaceutically acceptable salt or conjugate thereof, one or more glucocorticoid or a therapeutically active analogue, derivative, homolog, pharmaceutically acceptable salt or conjugate thereof, an antileukotrine and/or a leukotriene receptor antagonist. The COX inhibitor may inhibit one or both of COX-1 and COX-2. The COX inhibitor may comprise a Non-Steroidal Anti-Inflammatory Drug (NSAID). The NSAID may comprise ibuprofen, copper ibuprofenate, indomethacin, copper indomethacin, naproxen, flurbiprofen and/or celecoxib.

The one or more anti-inflammatory may for example comprise one or more of: aceclofenac, acemetacin, acetylsalicylic acid, 5-amino-acetylsalicylic acid, alclofenac, alminoprofen, amfenac, bendazac, bermoprofen, alpha-bisabolol, bromfenac, bromosaligenin, bucloxic acid, butibufen, carprofen, cinmetacin, clidanac, clopirac, diclofenac sodium, diflunisal, ditazol, enfenamic acid, etodolac, etofenamate, felbinac, fenbufen, fenclozic acid, fendosal, fenoprofen, fentiazac, fepradinol, flufenamic acid, flunixin, flunoxaprofen, flurbiprofen, glucametacin, glycol salicylate, ibuprofen, ibuproxam, indomethacin, indoprofen, isofezolac, isoxepac, isoxicam, ketoprofen, ketorolac, lornoxicam, loxoprofen, meclofenamic acid, mefenamic acid, meloxicam, mesalamine, metiazinic acid, mofezolac, naproxen, niflumic acid, oxaceprol, oxaprozin, oxyphenbutazone, parsalmide, perisoxal, phenyl acetylsalicylate, olsalazine, pyrazolac, piroxicam, pirprofen, pranoprofen, protizinic acid, salacetamide, salicilamide O-acetic acid, salicylsulphuric acid, salsalate, sulindac, suprofen, suxibuzone, tenoxicam, tiaprofenic acid, tiaramide, tinoridine, tolfenamic acid, tolmetin, tropesin, xenbucin, ximoprofen, zaltoprofen, zomepirac, tomoxiprol; and sulindac.

The one or more anti-inflammatory may comprise one or more mineralocorticoid and/or one or more glucocorticoid or a therapeutically active analogue, derivative, homolog, pharmaceutically acceptable salt or conjugate thereof.

The one or more mineralocorticoid or a therapeutically active analogue, derivative, homolog, pharmaceutically acceptable salt or conjugate thereof may comprise one or more of: 11-desoxycortisone (11-DC); fludrocortisone; fludrocortisone acetate (FA); fludrocortisone acetonide; Deoxycorticosterone acetate (DA); Deoxycorticosterone (DS); or Aldosterone; or a therapeutically active analogue, derivative, homolog, pharmaceutically acceptable salt or conjugate thereof.

The one or more glucocorticoid or a therapeutically active analogue, derivative, homolog, pharmaceutically acceptable salt or conjugate thereof may comprise one or more of: cortisol, cortisone, prednisone, prednisolone, methylprednisolone, dexamethasone, betamethasone, triamcinolone, triamcinolone acetonide, beclometasone or a therapeutically active analogue, derivative, homolog, pharmaceutically acceptable salt or conjugate thereof.

The one or more mineralocorticoid and/or more glucocorticoid or a therapeutically active analogue, derivative, homolog, pharmaceutically acceptable salt or conjugate thereof may comprise one or more dual action compounds, wherein each dual action compound is capable of modulating the activity of both a mineralocorticoid receptor and a glucocorticoid receptor.

The dual action compound may comprise one or more of triamcinolone; triamcinolone acetonide; cortisol; cortisone; prednisone; prednisolone; methylprednisolone; fludrocortisone; fludrocortisone acetate; fludrocortisone acetonide; or a therapeutically active analogue, derivative, homolog, pharmaceutically acceptable salt or conjugate thereof.

According to the present invention the one or more mineralocorticoid or one or more glucocorticoid or a pharmaceutically acceptable salt or conjugate thereof comprises fludrocortisone or a pharmaceutically acceptable salt or conjugate thereof. The pharmaceutically acceptable salt or conjugate thereof comprises one or more of fludrocortisone acetate and fludrocortisone acetonide.

The fludrocortisone or a pharmaceutically acceptable salt or conjugate thereof.

In one particular embodiment the one or more mineralocorticoid and/or one or more glucocorticoid or a pharmaceutically acceptable salt or conjugate thereof comprises triamcinolone acetonide or a acceptable salt or conjugate thereof.

When the one or more mineralocorticoid and/or one or more glucocorticoid or a pharmaceutically acceptable salt or conjugate thereof comprises triamcinolone acetonide the concentration may comprise 3.0 to 5.0 mg/ml. In one particular embodiment the concentration may comprise 4.0 mg/ml.

In another particular embodiment, the one or more mineralocorticoid and or one or more glucocorticoid or a pharmaceutically acceptable salt or conjugate thereof comprises triamcinolone and fludrocortisone or a pharmaceutically acceptable salt or conjugate of either. The pharmaceutically acceptable salt or conjugate thereof may comprise one or more of fludrocortisone acetate and fludrocortisone acetonide and triamcinolone acetonide.

In another embodiment, the one or more mineralocorticoid and/or one or more glucocorticoid or a pharmaceutically acceptable salt or conjugate thereof comprises a mixture of one or more mineralocorticoid or a pharmaceutically acceptable salt or conjugate thereof and one or more glucocorticoid or a pharmaceutically acceptable salt or conjugate thereof. The mixture may comprise: two or more mineralocorticoids or a pharmaceutically acceptable salt or conjugate thereof; two or more glucocorticoids or a acceptable salt or conjugate thereof and/or one or more mineralocorticoid or a pharmaceutically acceptable salt or conjugate thereof and one or more glucocorticoids or a pharmaceutically acceptable salt or conjugate thereof.

In one embodiment the pharmaceutical composition is injected into the eye. The injection may comprise suprachoroidal injection.

In another embodiment, the at least one anti-inflammatory is provided in a unit-dose formulation. The unit dose formulation may be provided in a pre-filled syringe. The pre-filled syringe may comprise two barrels. A first barrel may comprise the pharmaceutical composition of the second or third aspects. A second barrel may comprise one or more additional agent.

The one or more pharmaceutically acceptable carriers, diluents or excipients may comprise one or more surfactant or wetting agent.

The surfactant may comprise a polysorbate. The polysorbate may comprise one or more of polysorbate 20 and polysorbate 80. In a particular embodiment the surfactant comprises polysorbate 80.

The pharmaceutically acceptable carrier, diluent or excipient may comprise carboxy methyl cellulose (CMC).

The pharmaceutical composition further comprises one or more of a pH adjustment composition and water for injection. The pH adjustment composition may comprise hydrochloric acid and/or sodium hydroxide.

The pharmaceutical composition may comprise a pH from 6 to 8. The pH may comprise from 6 to 7.5.

The pharmaceutical composition may comprise a balanced salt solution. The balanced salt solution may comprise a saline and a buffer. The balanced salt solution one or more of sodium chloride; potassium chloride; calcium chloride (dehydrate); magnesium chloride (hexahydrate); sodium acetate (trihydrate); sodium citrate (dehydrate); hydrochloric acid; sodium hydroxide and water for injection.

In another particular embodiment, the pharmaceutical composition is preservative free.

According to any one of the above aspects, the pharmaceutical composition of the invention may comprise a sustained release composition.

In a particular embodiment, the pharmaceutical composition or one or more component thereof may be sterilized.

Dry AMD comprises early AMD and geographic atrophy (GA), distinct from exudative AMD. Distinct from exudative AMD means exudative AMD is not comprised within dry AMD.

In another embodiment, treatment comprises prophylactic treatment. The prophylactic treatment may comprise treatment to prevent dry AMD, treatment of a predisposition to dry AMD or treatment of a susceptibility to dry AMD. The predisposition or susceptibility may comprise an individual with a family history of an eye disease or condition such as, dry AMD. Prevention or prophylaxis may be successful if the development of dry AMD is completely or partially prevented or slowed down.

In one embodiment of any one of the above aspects, the pharmaceutical composition further comprises at least one additional agent.

The at least one additional agent may comprise an anti-VEGF (anti-Vascular Endothelial Growth Factor). The anti-VEGF may comprise one or more of ranibizumab (brand name Lucentis^{®}); aflibercept (brand name Eylea^{®}); bevacizumab (brand name Avastin^{®}) and OPT-302.

Further aspects and/or features of the present invention will become apparent from the following detailed description.

### BRIEF DESCRIPTION OF THE DRAWINGS

In order that the invention may be readily understood and put into practical effect, reference will now be made to embodiments of the present invention with reference to the accompanying drawings, wherein like reference numbers refer to identical elements. The drawings are provided by way of example only, wherein:

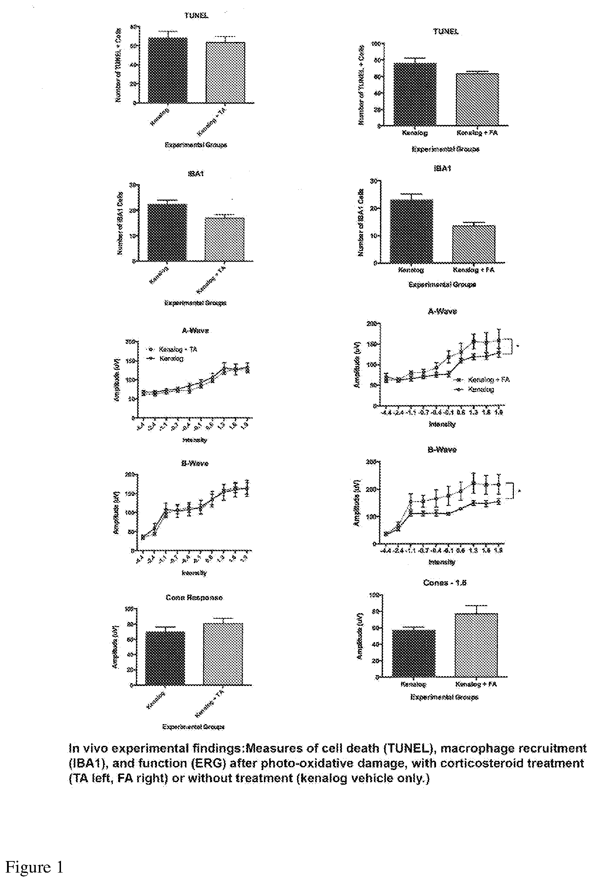

Figure 1: *In vivo* experimental findings: measures of cell death (Terminal deoxynucleotidyl transferase dUTP nick end labelling; TUNEL), macrophage recruitment (IBA1), and function (ERG) after photo-oxidative damage, with corticosteroid treatment (TA left, FA right) or without treatment (Kenelog vehicle only).

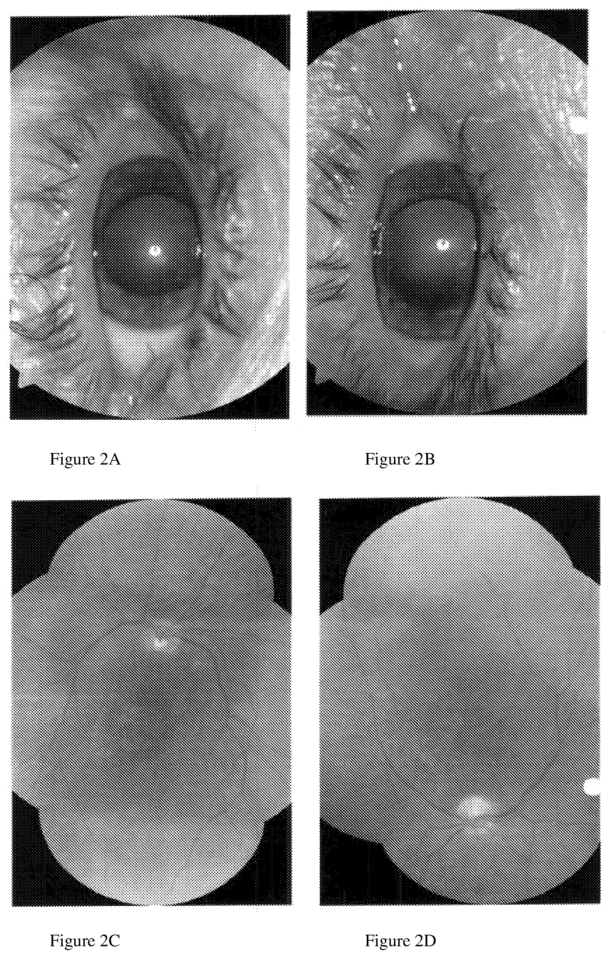

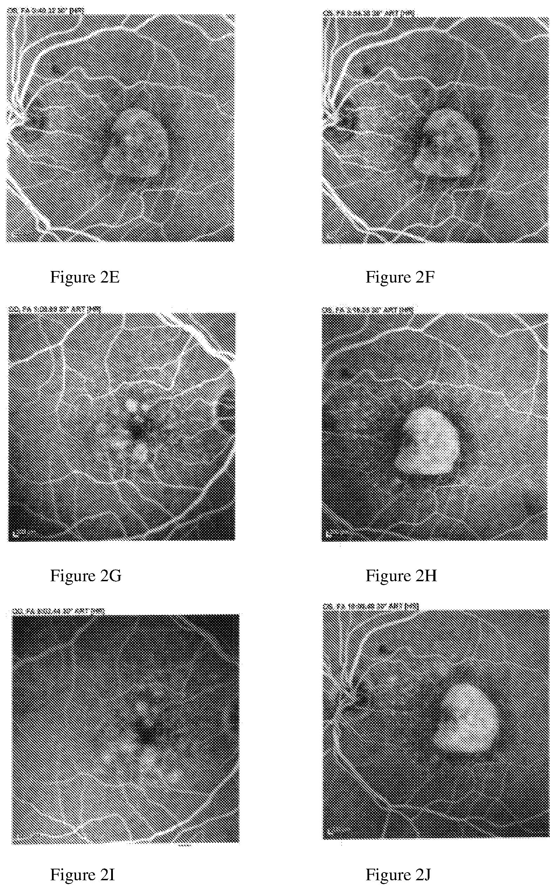

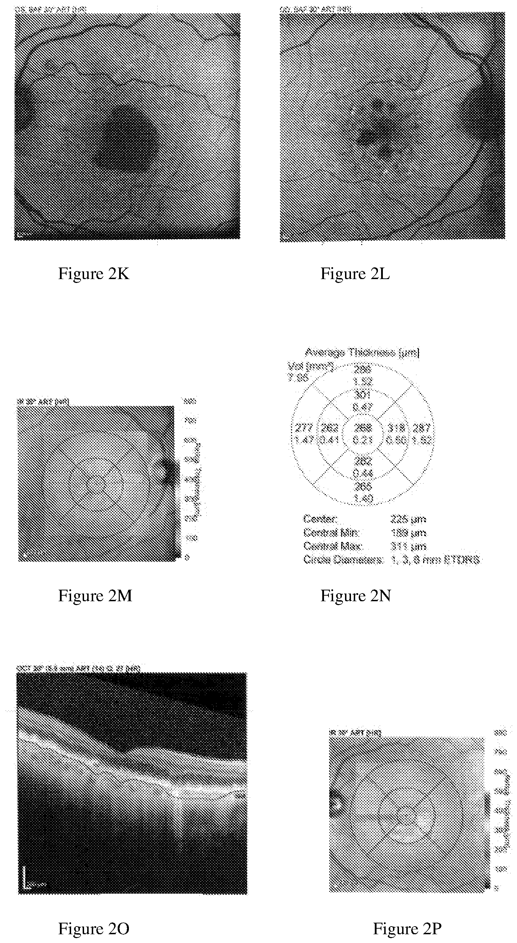

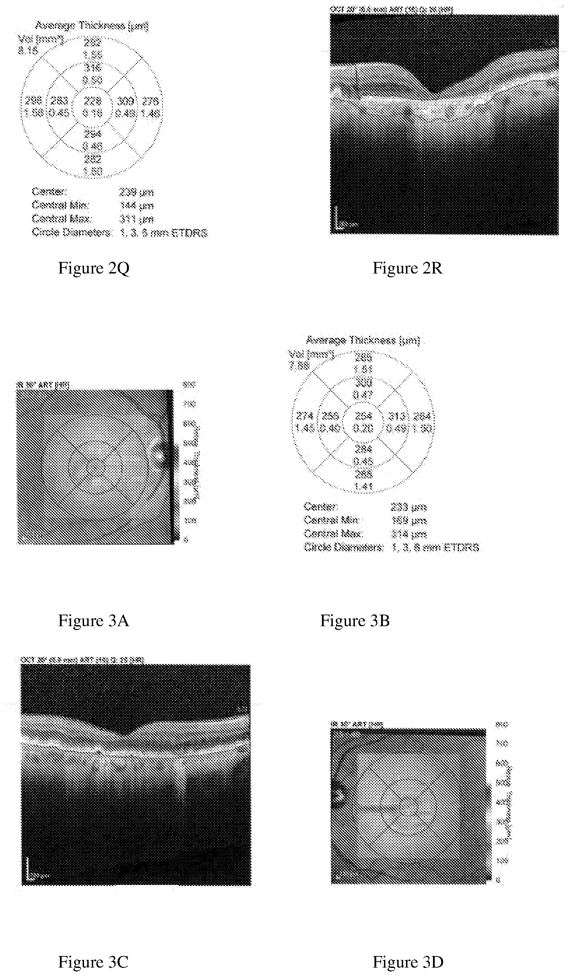

Figure 2: shows data collected on a subject at initial screening for proposed injection with fludrocortisone according to one embodiment of the invention. Figures 2A and 2B show photographs of the left and right eye of the subject (FCA-001). Figures 2B and 2C show retinographs of the same eyes. Figures 2E to 2J show tracking laser tomography of the same eyes: OS (oculus sinister; left eye), FA 0:40.32 30° [HR] (Figure 2E); OS, FA 0:54.38 30° ART [HR] (Figure 2F); OD (oculus dextrus; right eye), FA 1:08.69 30° ART[HR] (Figure 2G); OS, FA 3:15.35 30° ART [HR] (Figure 2H); OD, FA 5:02.44 30° ART[HR] (Figure 2I); OS, FA 10:09.49 30°[HS] (Figure 2J). Figures 2K and 2L show further tracking tomography images: OS, BAF 30° ART[HR] (Figure 2K); OD, BAF 30° ART[HR] (Figure 2L). Figures 2M to 2R show thickness mapping of the same eyes: OD IR 30° ART [HR] (Figures 2M and 2N); OD OCT 20° (5.9 mm) ART (14) A: 27 [HR] (Figure 2O); OS IR 30° ART[HR] (Figures 2P and 2Q); OS OCT 20° (5.5 mm) ART (16) Q: 26[HR] (Figure 2R).

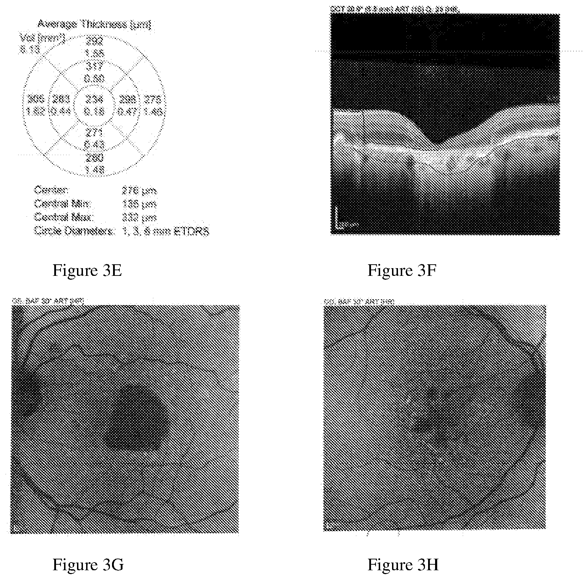

Figure 3: shows data collected on the same subject at Day 0, *i.e.* before treatment. Figures 3A to 3F show tracking laser tomography: OD IR 30° ART[HR] (Figures 3A and 3B); OD OCT 20° (5.9 mm) ART (16) Q: 25[HR] (Figure 3C); OS IR 30° ART[HR] (Figures 3D and 3E); OS OCT 20.0° (5.5 mm) ART (16) Q: 29 [HR] (Figure 3F). Figures 3G and 3H show tracking tomography of the same eyes: OS, BAF 30° ART[HR] (Figure 3G) and OD, BAF 30° ART[HR] (Figure 3H).

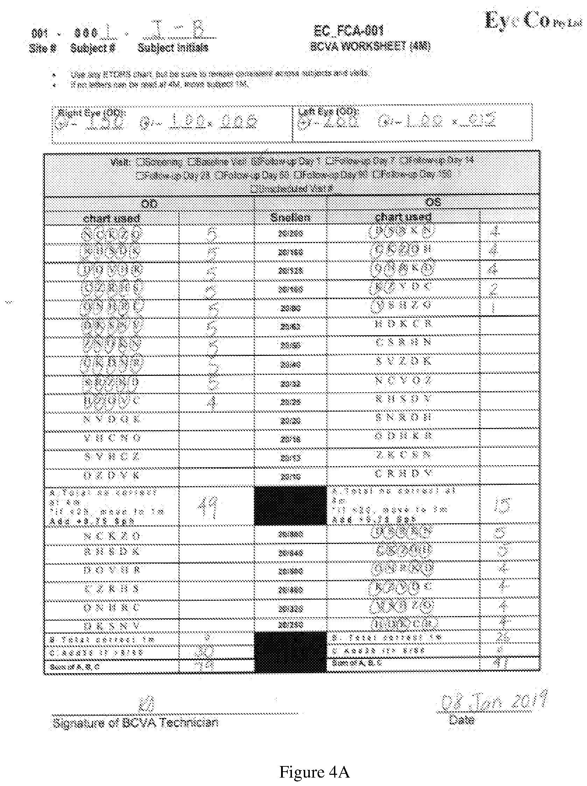

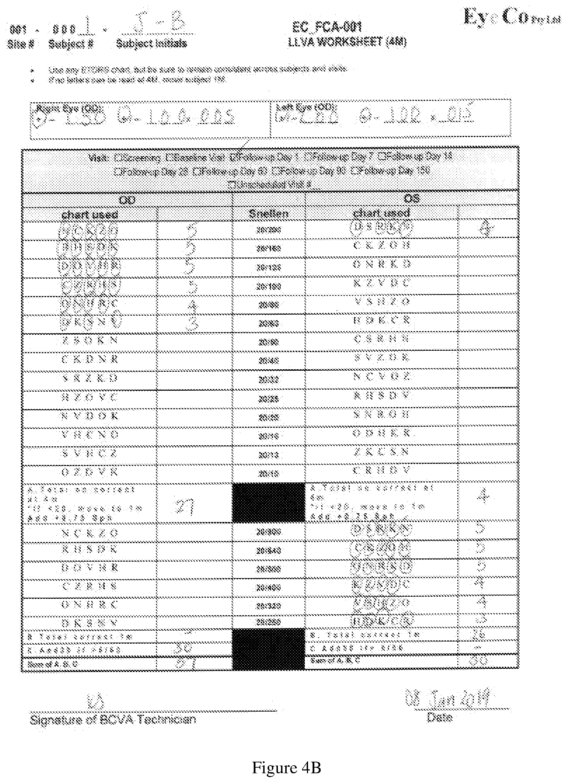

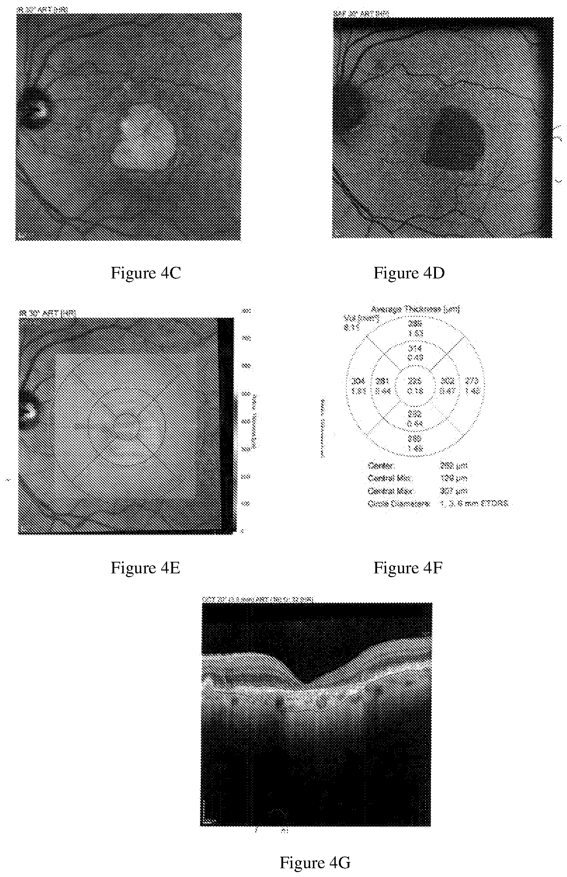

Figure 4: shows data collected on the same subject at Day 1, *i.e.* after injection with fludrocortisone according to one embodiment of the invention. Figures 4A and 4B show eye tests (Snellan charts). Figures 4C and 4D show tracking laser tomography images: OS IR 30° ART[HR] (Figure 4C); and OS BAF 30° ART[HR] (Figure 4D). Figures 4E to 4G show tracking laser tomography: OS IR 30° ART[HR] (Figures 4E and 4F); and OCT 20° (5.5 mm) ART (16) Q: 32[HR] (Figure 4G).

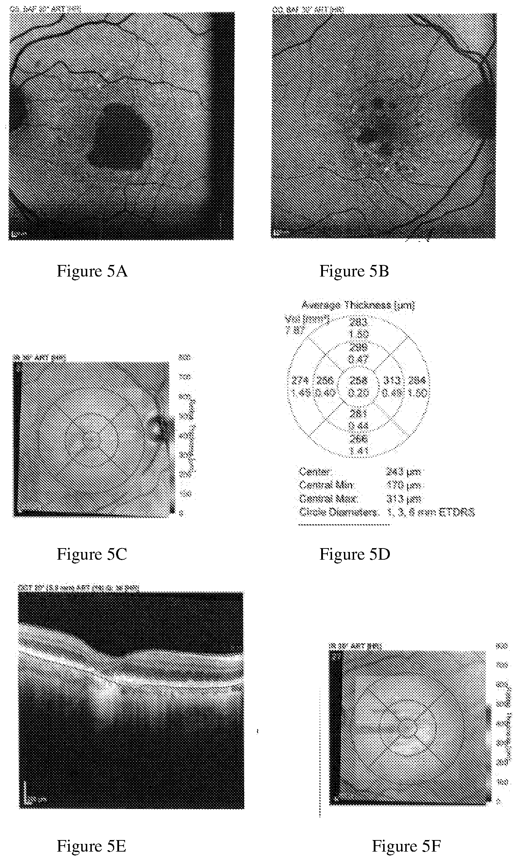

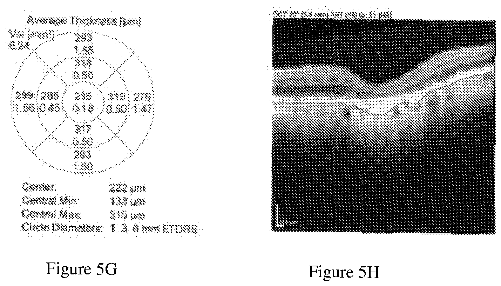

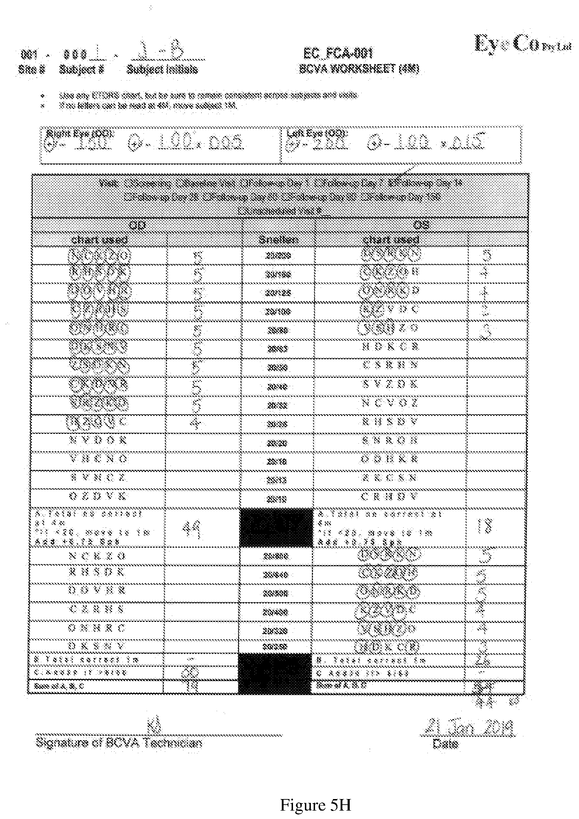

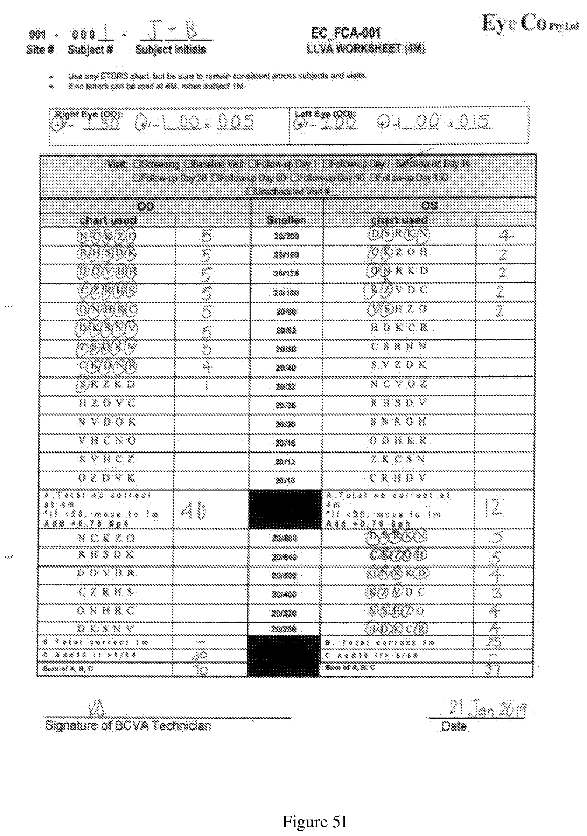

Figure 5: shows data collected from the same subject at Day 14. Figures 5A and 5B show tracking laser tomography: OS, BAF 30° ART[HR] (Figure 5A); OD, BAF 30° ART[HR] (Figure 5B). Figures 5C to 5H show further laser tomography images: OD IR 30 ART[HR] (Figures 5C and 5D); OD OCT 20° (5.9 mm) ART (16) Q: 30[HR] (Figure 5E); OS IR 30° ART[HR] (Figures 5F and 5G); OS OCT 20° (5.5 mm) ART (16): Q 31[HR] (Figure 5H). Figure 5H and 5I show eye tests (Snellan charts).

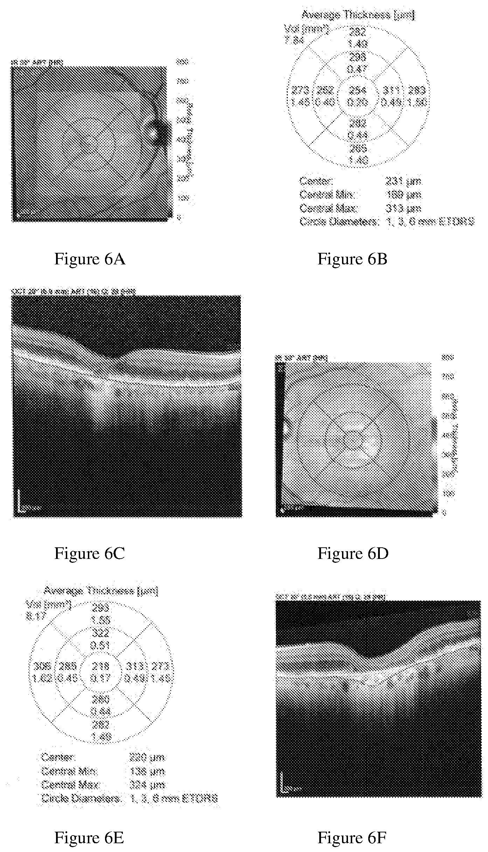

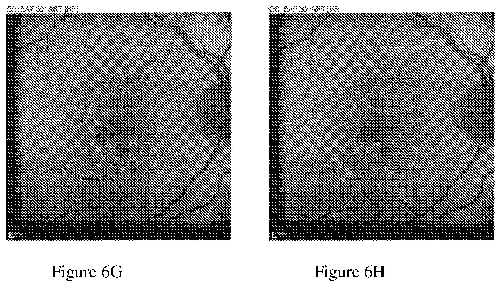

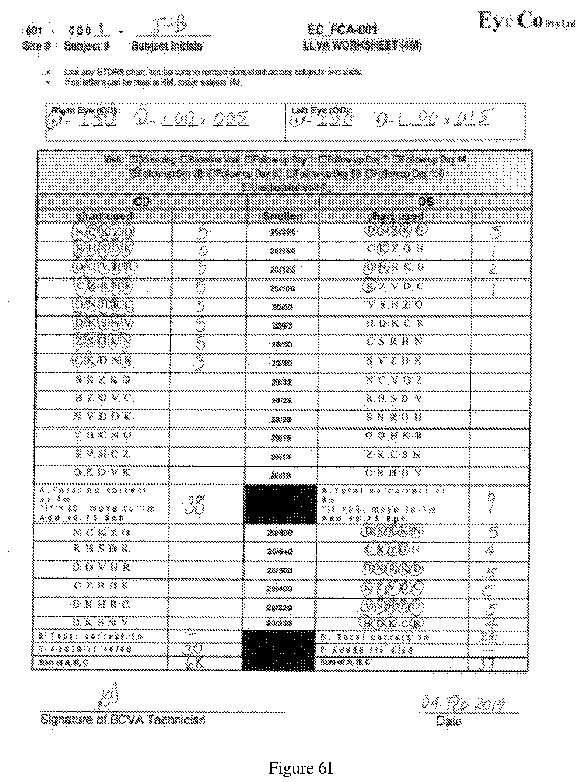

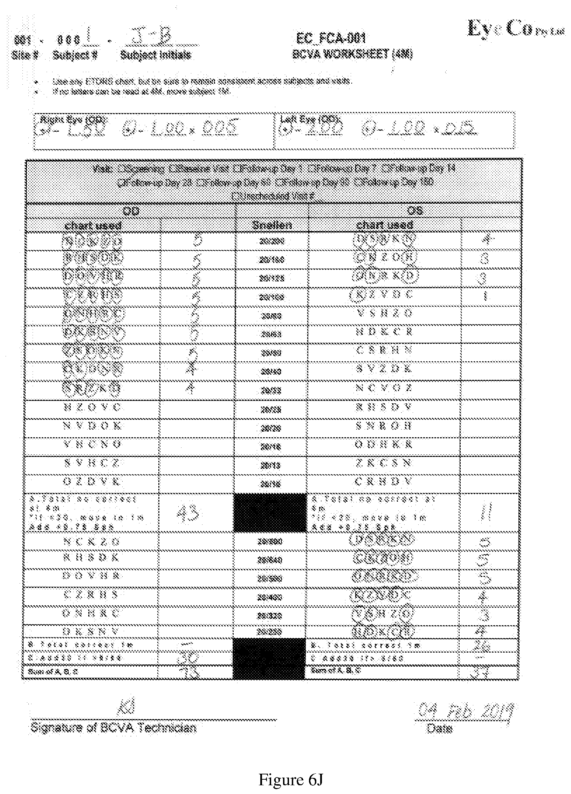

Figure 6: shows data collected from the same subject at Day 28. Figures 6A to 6H show tracking laser tomography results: OD IR 30° ART[HR] (Figure 6A and 6B); OD OCT 20° (5.9 mm) ART (16) Q: 28[HR] (Figure 6C). OS IR 30° ART[HR] (Figures 6D and 6E); OCT 20° (5.5 mm) ART(16) Q: 29[HR] (Figure 6F); OD, BAF 30° ART[HR] (Figure 6G); OS, BAF 30° ART[HR] (Figure 6H). Figure 6H and 6I show eye tests (Snellan charts).

Skilled addressees will appreciate that elements in the drawings are illustrated for simplicity and clarity and have not necessarily been drawn to scale. For example, the relative dimensions of some elements in the drawings may be distorted to help improve understanding of embodiments of the present invention.

### DETAILED DESCRIPTION OF THE INVENTION

Embodiments of the present invention relate to a composition for use in the treatment of geographic atrophy. More particularly, this invention relates to a composition used as defined in claim 1.

Dry AMD is a medical condition which may result in blurred or no vision in the center of the visual field. While in the early stages of this disease, the progression sees a gradual worsening of vision that may affect one or both eyes. Although it does not cause complete blindness, the resultant loss of central vision can make it difficult to recognize faces, drive, read, perform other daily activities and can reduce quality of life.

As used herein Dry AMD includes Geographic Atrophy (GA). GA may also be known as atrophic age-related macular degeneration (AMD) or advanced dry AMD, which is an advanced form of age-related macular degeneration that can result in the progressive and irreversible loss of retina (photoreceptors, RPE and choriocappillaris).

Herein *"dry AMD"* is used to refer to dry AMD, GA and atrophic AMD. As used herein dry AMD comprises early AMD and geographic atrophy (GA), distinct from exudative AMD.

Currently, more vision is lost to the dry form of AMD than the wet form. A long felt want exists for a treatment for dry AMD.

The inventor recognises that the dry lesion of dry AMD is regarded as the natural end stage of Macular Degeneration in the absence of neovascularisation. This means that in a significant number of cases the eye is dry rather than wet.

The data presented herein generated on a rodent (both mouse and rat models exist) model of dry AMD, which is published and recognised. The model is recognised and a number of therapeutic interventions have been accomplished in the model and show the model is pertinent and maps onto a human model and/or has parallels with the human condition).

While not wanting to be bound by any one theory, the inventor hypothesises that in dry AMD macrophages enter a cleavage plane between the basement membrane of the RPE and the Brook's membrane, which includes the basement membrane of the choroid, and resulting inflammation peels back the RPE.

In many instances the chronic inflammatory cells become established between the basement membrane of the RPE and the choroid. Because a low grade chronic inflammatory process exacerbates and expands the GA lesion (the natural end stage of dry AMD in the absence of neovascularistion), the inventor realises that fludrocortisone is indicated for end stage dry AMD along with TA or other anti-inflammatories.

For this reason the inventor has proposed that dry-AMD may be effectively treated with an anti-inflammatory, like fludrocortisone or also triamcinolone. A clinician could inject a patient with fludrocortisone that has rapidly advancing dry AMD.

Also disclosed is a method of treating dry AMD in a subject , the method comprising administering to the subject a therapeutically effective amount of an anti-inflammatory to thereby treat the dry AMD.

The invention provides a pharmaceutical composition used as defined in the claims.

The use of a pharmaceutical composition comprising one or more anti-inflammatory for the manufacture of a medicament for the treatment of dry AMD is also disclosed herein.

The one or more anti-inflammatory may comprise one or more of a COX inhibitor, one or more mineralocorticoid or a pharmaceutically acceptable salt or conjugate thereof, one or more glucocorticoid or a pharmaceutically acceptable salt or conjugate thereof, an antileukotrine and/or a leukotriene receptor antagonist. The COX inhibitor may inhibit one or both of COX-1 and COX-2. The COX inhibitor may comprise a Non-Steroidal Anti-Inflammatory Drug (NSAID). The NSAID may comprise ibuprofen, copper ibuprofenate, indomethacin, copper indomethacin, naproxen, flurbiprofen and/or celecoxib.

The one or more anti-inflammatory may for example comprise one or more of: aceclofenac, acemetacin, acetylsalicylic acid, 5-amino-acetylsalicylic acid, alclofenac, alminoprofen, amfenac, bendazac, bermoprofen, alpha-bisabolol, bromfenac, bromosaligenin, bucloxic acid, butibufen, carprofen, cinmetacin, clidanac, clopirac, diclofenac sodium, diflunisal, ditazol, enfenamic acid, etodolac, etofenamate, felbinac, fenbufen, fenclozic acid, fendosal, fenoprofen, fentiazac, fepradinol, flufenamic acid, flunixin, flunoxaprofen, flurbiprofen, glucametacin, glycol salicylate, ibuprofen, ibuproxam, indomethacin, indoprofen, isofezolac, isoxepac, isoxicam, ketoprofen, ketorolac, lornoxicam, loxoprofen, meclofenamic acid, mefenamic acid, meloxicam, mesalamine, metiazinic acid, mofezolac, naproxen, niflumic acid, oxaceprol, oxaprozin, oxyphenbutazone, parsalmide, perisoxal, phenyl acetylsalicylate, olsalazine, pyrazolac, piroxicam, pirprofen, pranoprofen, protizinic acid, salacetamide, salicilamide O-acetic acid, salicylsulphuric acid, salsalate, sulindac, suprofen, suxibuzone, tenoxicam, tiaprofenic acid, tiaramide, tinoridine, tolfenamic acid, tolmetin, tropesin, xenbucin, ximoprofen, zaltoprofen, zomepirac, tomoxiprol; and sulindac.

The one or more anti-inflammatory may comprise one or more mineralocorticoid and/or one or more glucocorticoid or a therapeutically active analogue, derivative, homolog, pharmaceutically acceptable salt or conjugate thereof.

The one or more mineralocorticoid or a therapeutically active analogue, derivative, homolog, pharmaceutically acceptable salt or conjugate thereof may comprise one or more of: 11-desoxycortisone (11-DC); fludrocortisone; fludrocortisone acetate (FA); fludrocortisone acetonide; Deoxycorticosterone acetate (DA); Deoxycorticosterone (DS); or Aldosterone; or a pharmaceutically acceptable salt or conjugate thereof.

The one or more glucocorticoid or a pharmaceutically acceptable salt or conjugate thereof may comprise one or more of: cortisol, cortisone, prednisone, prednisolone, methylprednisolone, dexamethasone, betamethasone, triamcinolone, triamcinolone acetonide, beclometasone or a pharmaceutically acceptable salt or conjugate thereof.

The one or more mineralocorticoid and/or one or more glucocorticoid or a pharmaceutically acceptable salt or conjugate thereof may comprise one or more dual action compounds, wherein each dual action compound is capable of modulating the activity of both a mineralocorticoid receptor and a glucocorticoid receptor.

The dual action compound may comprise one or more of triamcinolone, triamcinolone acetonide; cortisol; cortisone; prednisone; prednisolone; methylprednisolone; fludrocortisone; fludrocortisone acetate; fludrocortisone acetonide or pharmaceutically acceptable salt or conjugate thereof.

According to the present invention the one or more mineralocorticoid or one or more glucocorticoid or a pharmaceutically acceptable salt or conjugate thereof comprises fludrocortisone or a acceptable salt or conjugate thereof. The pharmaceutically acceptable salt or conjugate thereof comprises one or more of fludrocortisone acetate and fludrocortisone acetonide.

The one or more mineralocorticoid and/or one or more glucocorticoid or a pharmaceutically acceptable salt or conjugate thereof may comprise triamcinolone acetonide or a acceptable salt or conjugate thereof. When the one or more mineralocorticoid and/or one or more glucocorticoid or a pharmaceutically acceptable salt or conjugate thereof comprises triamcinolone acetonide the concentration may comprise 3.0 to 5.0 mg/ml. In one particular embodiment the concentration may comprise 4.0 mg/ml.

From the teaching herein a skilled person is readily able to select suitable dosages for the one or more anti-inflammatory.

The one or more mineralocorticoid and or one or more glucocorticoid or a pharmaceutically acceptable salt or conjugate thereof may comprise triamcinolone and fludrocortisone or a pharmaceutically acceptable salt or conjugate of either. The pharmaceutically acceptable salt or conjugate thereof may comprise one or more of fludrocortisone acetate and fludrocortisone acetonide and triamcinolone acetonide.

The one or more mineralocorticoid and/or one or more glucocorticoid or a pharmaceutically acceptable salt or conjugate thereof may comprise a mixture of one or more mineralocorticoid or a pharmaceutically acceptable salt or conjugate thereof and one or more glucocorticoid or a pharmaceutically acceptable salt or conjugate thereof. The mixture may comprise: two or more mineralocorticoids or a pharmaceutically acceptable salt or conjugate thereof; two or more glucocorticoids or a pharmaceutically acceptable salt or conjugate thereof and/or one or more mineralocorticoid and one or more glucocorticoids or a pharmaceutically acceptable salt or conjugate thereof.

The at least one anti-inflammatory may be injected into the eye. The injection may comprise suprachoroidal injection.

The at least one anti-inflammatory may be provided in a unit-dose formulation. The unit dose formulation may be provided in a pre-filled syringe. The pre-filled syringe may comprise two barrels. A first barrel may comprise the pharmaceutical composition of the second or third aspects. A second barrel may comprise one or more additional agent.

As used herein, the term "unit dose" is used to refer to a pharmaceutical composition in the form in which it is to be used. The unit dose may comprise the pharmaceutical composition of the invention in a particular dose; volume; particle size; pH; viscosity; and/or degree of flocculation. The unit dose may comprise the non-reusable packaging such as, the syringe or double-barrelled syringe.

The one or more pharmaceutically acceptable carriers, diluents or excipients may comprise one or more surfactant or wetting agent.

The surfactant may comprise a polysorbate. The polysorbate may comprise one or more of polysorbate 20 and polysorbate 80. In a particular embodiment the surfactant comprises polysorbate 80.

The pharmaceutically acceptable carrier, diluent or excipient may comprise carboxy methyl cellulose (CMC).

The pharmaceutical composition may further comprise one or more of a pH adjustment composition and water for injection. The pH adjustment composition may comprise hydrochloric acid and/or sodium hydroxide.

The pharmaceutical composition may comprise a pH from 6 to 8. The pH may comprise from 6 to 7.5.

The pharmaceutical composition may comprise a balanced salt solution. The balanced salt solution may comprise a saline and a buffer. The balanced salt solution may comprise may comprise one or more of sodium chloride; potassium chloride; calcium chloride (dehydrate); magnesium chloride (hexahydrate); sodium acetate (trihydrate); sodium citrate (dehydrate); hydrochloric acid; sodium hydroxide and water for injection.

The pharmaceutical composition may be preservative free.

The pharmaceutical composition of the invention may comprise a sustained release composition.

The pharmaceutical composition of the invention may further comprise at least one additional agent.

The at least one additional agent may comprise an anti-VEGF (anti-Vascular Endothelial Growth Factor). The anti-VEGF may comprise one or more of ranibizumab (brand name Lucentis^{®}); aflibercept (brand name Eylea^{®}); bevacizumab (brand name Avastin^{®}) and OPT-302.

The pharmaceutical composition may comprise carboxymethyl cellulose (CMC).

The pharmaceutical composition may comprise a surfactant. As used herein, the term *"surfactant"* refers to any agent, which preferentially absorbs to an interface between two immiscible phases, such as the interface between water and an organic polymer solution, a water/air interface or organic solvent/air interface. Surfactants generally possess a hydrophilic moiety and a lipophilic moiety; such that, upon absorbing to microparticles, they tend to present moieties to the external environment that do not attract similarly coated particles, thus reducing particle agglomeration. Surfactants may also promote absorption of a therapeutic or diagnostic agent and increase bioavailability of the agent.

The surfactant may comprise a polysorbate, such as one or more of polysorbate 20 and polysorbate 80. In a preferred embodiment, the surfactant comprises polysorbate 80.

*"Prevention"* or *"prophylaxis,"* as used herein, refers to prophylactic or preventative measures. Those in need of prevention or prophylaxis include those in whom the dry AMD is to be prevented, and in some embodiments, may be predisposed or susceptible to the eye disease or condition e.g. individuals with a family history of an eye disease or condition.

Prevention or prophylaxis is successful herein if the development of dry AMD is completely or partially prevented or slowed down.

*"Treatment"* of a subject herein refers to therapeutic treatment. Those in need of treatment include those already with dry AMD, as well as those in whom the progress of dry AMD is to be prevented. Hence, the subject may have been diagnosed as having dry AMD or may have dry AMD or damage that is likely to progress in the absence of treatment. Alternatively, the subject may be symptom-free, but has risk factors for development of dry AMD e.g., positive family history. Treatment is successful herein if the dry AMD is alleviated or healed, or progression of the dry AMD, including its signs and symptoms and/or structural damage, is halted or slowed down as compared to the condition of the subject prior to administration. Successful treatment further includes complete or partial prevention of the development of the dry AMD. For purposes herein, slowing down or reducing the dry AMD or the progression of the dry AMD is the same as arrest, decrease, or reversal of the dry AMD.

The expression *"effective amount"* refers to an amount of an agent or medicament, either in a single dose or as part of a series, which is effective for treating or preventing dry AMD or predisposition thereto. This would include an amount that is effective in achieving a reduction in one or more symptom as compared to baseline prior to administration of such amount as determined, e.g., by visual acuity or other testing. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

The terms *"subject", "patient"* or *"individual,"* which are used interchangeably herein, refer to any subject, particularly a vertebrate subject, and even more particularly a mammalian subject, for whom therapy or prophylaxis is desired. Suitable vertebrate animals that fall within the scope of the invention include, but are not restricted to, any member of the subphylum Chordata including humans, as well as non-human primates, rodents (e.g., mice rats, guinea pigs), lagomorphs (e.g., rabbits, hares), bovines (e.g., cattle), ovines (e.g., sheep), caprines (e.g., goats), porcines (e.g., pigs), equines (e.g., horses), canines (e.g., dogs), felines (e.g., cats), avians (e.g., chickens, turkeys, ducks, geese, companion birds such as canaries, budgerigars etc.), marine mammals (e.g., dolphins, whales), reptiles (snakes, frogs, lizards etc.), and fish. In specific embodiments, the "subject", "patient" or "individual" is a human in need of treatment or prophylaxis of an eye disease or condition, including in subjects with a diabetic eye disease or condition or an ocular tumour. In specific embodiments, the terms "subject", "patient" or "individual" refer to any single human subject, including a patient, eligible for treatment who is experiencing or has experienced one or more signs, symptoms, or other indicators of dry AMD or predisposition thereto, whether, for example, newly diagnosed or previously diagnosed and now experiencing a recurrence or relapse, or is at risk for dry AMD, no matter the cause. Intended to be included as a "subject", "patient" or "individual" are any subjects involved in clinical research trials not showing any clinical sign of disease, or subjects involved in epidemiological studies, or subjects once used as controls. The "subject", "patient" or "individual" may have been previously treated with a medicament for dry AMD, or not so treated.

As used herein, glucocorticoid and mineralocorticoid includes a therapeutically active analog, derivative, pharmaceutically acceptable salt, prodrug, metabolite or conjugate thereof.

As used herein, a derivative includes a therapeutically active or pharmaceutically active fragment of a compound modulating the activity of a mineralocorticoid receptor or a glucocorticoid receptor.

An analog may be a structural analog or a functional analog.

A homolog may comprise a molecule of the same chemical type, but differing by a fixed increment of an atom or a constant group of atoms. An example is methyl and ethyl alcohols which are homologous.

Table 1 below shows some example compounds and their measured mineralocorticoid and glucocorticoid potencies.

In one embodiment, the compositions of the invention comprise a sustained release composition. Based on the teachings herein, a skilled person is readily able to select and/or formulate a suitable sustained release composition.

In another embodiment, the compositions and components thereof may be sterilised. From the teachings herein, a skilled person is readily able to select a suitable sterilisation method such as, heat treatment.

In another embodiment, the compositions of the invention are preservative free.

In a particular embodiment, the compositions of the invention may be comprised in a syringe. In one embodiment, the syringe allows direct injection into an eye.

By "pharmaceutically-acceptable carrier, diluent or excipient" is meant a solid or liquid filler, diluent or encapsulating substance that may be safely used in systemic administration. Depending upon the particular route of administration, a variety of carriers, well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline and salts, such as mineral acid salts including hydrochlorides, bromides and sulfates, organic acids such as acetates, propionates and malonates and pyrogen-free water.

The one or more pharmaceutically acceptable carriers, diluents or excipients may comprise one or more of a wetting agent and a viscosity modifier.

A useful reference describing pharmaceutically acceptable carriers, diluents and excipients is Remington's Pharmaceutical Sciences (Mack Publishing Co. N.J. USA, 1991), which is incorporated herein by reference.

The above compositions may be administered in a manner compatible with the dosage formulation, and in such amount as is pharmaceutically-effective. The dose administered to a patient, in the context of the present invention, should be sufficient to effect a beneficial response in a patient over an appropriate period of time. The quantity of agent(s) to be administered may depend on the subject to be treated inclusive of the age, sex, weight and general health condition thereof, factors that will depend on the judgement of the practitioner.

The following non-limiting examples illustrate the invention. These examples should not be construed as limiting: the examples are included for the purposes of illustration only. The Examples will be understood to represent an exemplification of the invention.

### Examples

### METHODS

### Animals and Light Exposure:

All experiments conducted were in accordance with the ARVO Statement for the Use of Animals in Ophthalmic and Vision Research. Albino Sprague-Dawley (SD) rats aged from 130 to 160 postnatal days were exposed to bright continuous light (BCL) at 1000 lux . The rats were born and reared in dim cyclic light conditions (12 h light:12 h dark) with an ambient light level of approximately 5 lux. Exposure to BCL was conducted on animals aged between post-natal days (P) 90-150. Prior to BCL exposure, rats were dark adapted for a minimum of 15 h then transferred to individual cages designed to allow light to enter unimpeded. There were no areas of shadow in the cages; pupillary dilation was not performed. BCL exposure commenced consistently at 9:00 am, and was achieved using a cold-white fluorescent light source positioned above the cages (18W, Cool White; TFC), at an intensity of approximately 1000 lux at the cage floor. BCL exposure was maintained over a period of 24 h, after which time the animals were immediately returned to dim cyclic conditions for the post-exposure period. Animals were kept in dim light conditions following BCL exposure for a maximum period of 56 days.

The animals were exposed to BCL for a period of 1, 3, 6, 12, 17, or 24 hours, after which time retinal tissue was obtained for analysis. Some animals were returned to dimlight (5 lux) conditions immediately after 24 hours of BCL for a period of 3 or 7 days, to assess postexposure effects. Age-matched, dim-reared animals served as control samples.

### Tissue Collection and Processing

Animals were euthanatized by overdose of barbiturate administered by an intraperitoneal injection (60 mg/kg bodyweight; Valabarb; Virbac Animal Health, Regents Park, NSW, Australia). The left eye from each animal was marked at the superior surface for orientation and then enucleated and processed for cryosectioning, and the retina from the right eye was excised through a corneal incision and prepared for RNA extraction.

Eyes for cryosectioning were immediately immersion fixed in 4% paraformaldehyde in 0.1 M PBS (pH 7.3) for 3 hours at room temperature, then washed in 0.1M PBS before being left in a 15% sucrose solution overnight for cyroprotection. Eyes were oriented and embedded in O.C.T. compound (Tissue-Tek, Sakura, Japan) then snap frozen in liquid nitrogen and cryosectioned at 16 µm. Retinas for RNA extraction were immediately deposited in RNA stabilizer (RNAlater; Ambion, Austin, TX), prechilled on ice, and stored according to the manufacturer's instructions. RNA was then extracted from each sample , The samples were incubated at 4°C overnight to allow adequate penetration of the preservative, and then stored at 80°C until required. The samples were processed in batches encompassing the entire time course, to ensure comparability. On extraction, the retinal samples were thawed on ice, and the RNA stabilizer was removed. RNA extraction was performed with a combination of extraction reagent (TRIzol; cat. no. 15596-026; Invitrogen, Carlsbad, CA) and a purification kit (RNAqueous-Small Scale, cat. no. 1912; Ambion) used in tandem to extract and purify the RNA respectively, as described in another publication.

Isolated total RNA was analyzed for quantity and purity with a spectrophotometer (ND-1000; Nanodrop Technologies, Wilmington, DE), and samples with a 260/280 ratio greater than 1.90 were considered sufficient. The RNA quality in each sample was assessed (2100Bioanalyzer; Agilent Technologies, Santa Clara, CA) where only samples with an integrity number (RIN) of ≥8 were used.

### Microarray Experimentation and Analysis

Microarray analysis was performed using raw microarray data derived from a previous study (Natoli R, Zhu Y, Valter K, Bisti S, Eells J, Stone J. Gene and noncoding RNA regulation underlying photoreceptor protection: microarray study of dietary antioxidant saffron and photobiomodulation in rat retina. Mol Vis. 2010;16:1801-1822) using rat gene microarrays (Rat Gene 1.0 ST; Affymetrix, Santa Clara, CA). The full set of microarray data has been deposited in the NCBI Gene Expression Omnibus repository under accession number GSE22818 (National Center for Biotechnology Information, National Institutes of Health, Bethesda, MD). The analysis compared samples from dimreared and 24-hour BCL experimental groups (n ! 3 for each). The microarray data were analyzed (Partek Genomics Suite 6.4 software; Partek Inc., St. Louis, MO), and CEL files (Affymetrix) were imported into the software with background correction, normalization, and summarization, using the robust multiarray average (RMA) algorithm adjusted for probe sequence and GC content (GC-RMA). The processed values were displayed as individual probe sets representing exonic coding sequences, which were log-transformed using base 2. Differential expression analysis was performed using the analysis of variance (ANOVA) statistic with significance level of P " 0.05. The heterogeneity of the resulting differential expression data was evaluated with agglomerative hierarchical clustering, using the Euclidean distance metric and principle component analysis (PCA; both provided by the Genomics Suite; Partek). The differential expression data were then clustered according to biological process as described by the Gene Ontology Consortium, using functional analysis with Gene Ontology (GO) enrichment provided by the software (Partek GS Genomics Suite). After this, the list of differentially expressed genes was screened for those relating to the complement cascade, using a differential expression cutoff of #50% and aided by pathway information summarized from the Gene Ontology Consortium and gene grouping from the HUGO Gene Nomenclature Committee.

### Quantitative Real-Time Polymerase Chain Reaction

First-strand cDNA synthesis was performed as (SuperScript III Reverse Transcriptase kit, cat. no. 18080 - 044; Invitrogen) according to the manufacturer's instructions. A 20-µl. reaction mixture was used in conjunction with 1 µg RNA, 500 ng oligo (dT)18 primer, and 200 U reverse transcriptase.Gene amplification was measured using either commercially available hydrolysis probes (TaqMan; Applied Biosystems, Inc. [ABI], Foster City, CA) or SYBR Green with custom designed primers, the details of which are provided in Tables 2 and 3, respectively. The hydrolysis probes were applied according to a previously established qPCR protocol. The primers for SYBR Green qPCR (Table 3) were designed within a coding domain sequence transversing an intron using the Primer3 web-based design program (see Rosen *et al*.). The qPCR was performed using a commercial qPCR system (StepOnePlus; ABI). The amplification for each biological sample was performed in experimental triplicate, with the mean Cq (quantitation cycle) value then used to determine the ratio of change in expression. For both qPCRs (Taqman and SYBR Green; ABI), the percentage change compared to dim-reared samples was determined using the Cq method. The expression of the target gene was normalized to the expression of the reference gene glyceralde-hyde-3-phosphate dehydrogenase (GAPDH), which showed no differential expression in the present study or in previous light-induced retinal damage investigations. Amplification specificity was assessed using gel electrophoresis. Statistical analysis was performed using the one-way ANOVA, to assess the significance of the trend in expression. Differences with a P " 0.05 were considered statistically significant.

### In Situ Hybridization

To investigate the localization of C3 mRNA transcripts in the retina after BCL, a riboprobe to C3 was generated for in situ hybridization on retinal cryosections. C3 was cloned from a PCR product (483-bp amplicon) using cDNA prepared from rat retinas (as described above), the pGEM-T DNA vector system (Promega, Madison, WI), and TOP10 competent cells (One Shot; Invitrogen). A DIG RNA-labeling kit (SP6/T7; Roche, Basel, Switzerland) was used to transcribe linearized plasmid and generate DIG-labeled antisense and sense riboprobes. In situ hybridization was performed with a protocol described previously; the C3 riboprobe was hybridized overnight at 57°C, and then washed in saline sodium citrate (pH 7.4) at 60°C. After hybridization, some sections were further stained immunohistochemically (described later).

### Analysis of Cell Death

TUNEL labeling was used to quantify photoreceptor apoptosis in cryosections, during and after BCL, with a protocol published previously. Counts of TUNEL-positive cells in the outer nuclear layer (ONL) were performed along the full-length of retinal sections cut in the parasagittal plane (superioinferior), including the optic disc, in adjacent fields measuring 1000 % 1000 !m. The final count from each animal is the average at comparable locations in two nonsequential sections. Statistical analysis was performed by using one-way ANOVA. Differences with a P " 0.05 were considered statistically significant.

### Immunohistochemistry

Cryosections from each time point were used for immunohistochemical analysis, using primary antibodies for complement C3 (1:50; Abcam, Cambridge, MA), C3d (1:100; R&D Systems, Minneapolis, MN), ED1 (1:200; Millipore, Billerica, MA), and IBA1 (1:1000; Wako, Osaka, Japan). Immunohistochemistry was performed using a methodology previously described. Immunofluorescence was viewed with a laser scanning microscope (Carl Zeiss Meditec, Inc.) and acquired using PASCAL software (ver. 4.0; Carl Zeiss Meditec, Inc.). Images were enhanced for publication (Photoshop; Adobe, San Jose, CA), which was standardized between images.

### RESULTS

Figure 1 shows *in vivo* experimental findings: measures of cell death (TUNEL), macrophage recruitment (IBA1), and function (ERG) after photo-oxidative damage, with corticosteroid treatment (TA left, FA right) or without treatment (kenelog vehicle only).

The data in Figure 1 compares the efficacy of TA vs FA in reducing cell death, and preserving function in our light damage (photo-oxidative damage) model. The findings indicate that FA is more effective than TA in prevention of macrophage recruitment, and photoreceptor Death (TUNEL), and preserves retinal function. Data are consistent with the in vitro findings.

qPCR analyses of the retinas, as well as some histology images of the retinas, post treatment have also been performed.

### A Phase Ib, study of safety and tolerability of Intravitreal Fludrocortisone acetate (FA) and Triamcinolone (TA) in Patients with Geographic Atrophy (GA)

Investigational Product, Dose and Route of Administration: Fludrocortisone acetate (FA) 1mg/0.1ml & 2mg/0.1ml, Intravitreal (IVT) injection. The identical protocol will be followed with TA.

Rationale for dose: Single dose of 0.1 ml in a concentration of 1mg/0.1 mL and 2mg/0.1mL was selected based on a previous pre-clinical model. There were no signs of retinal toxicity on slit- lamp examination, indirect ophthalmoscopy, or by light microscopy in all eyes injected with 400ug/0.1mL, 1mg/0.1mL and 2mg/0.1mL. However, it was reported 1 case of intravitreal haemorrhage in 4 mg/0.1 mL.

Systemic injection of fludrocortisone acetate has serious mineral corticoid and glucocorticoid side effects such as hypertension, potassium loss, Cushingoid changes, etc. Intravitreal injection fludrocortisone acetate may avoid systemic side effects.

Objectives: To determine safety and tolerability of a single dose IVT injection of 1mg/0.1mL and 2mg/0.1mL FA in subjects with GA secondary to Age-Related Macular Degeneration (AMD).

Study Population: Study population will comprise of patients with GA secondary to AMD in both eyes with no previous treatment. The study is planned to enrol up to 9 participants.

Study Design: This study will enrol 9 participants to assess dose escalation based on a 3+3 algorithm. Enrolment will stop if > 2 patients experience dose-limiting toxicity at any time during the study. Dose-limiting toxicity is defined by intraocular inflammation, elevated IOP (intraocular pressure), reduced vision (loss of ≥15 letters), or haemorrhage within 28 days after injection.

Part 1 involves a single participant to assess safety and tolerability of 1mg/0.1mL FA. This participant will be followed for up to 28 days and reviewed by a safety review committee prior to the recruitment of a further 2 participants treated with 1mg/0.1mL FA totalling in 3 participants in the first cohort.

Part 2 involves a single participant to assess safety and tolerability of 2mg/0.1mL FA. This participant will be followed for up to 28 days and reviewed by an independent safety review committee prior to the recruitment of a further 5 participants treated with 2mg/0.1mL FA totalling in 6 participants in the second cohort.

The sample size (n=9) chosen for this study was selected without formal statistical justification, but the numbers chosen are considered adequate for assessing the study objectives. The sample size was determined on the basis of practical and logistical considerations for a pilot study, and not based on statistical power with regard to hypothesis testing or precision with regard to parameter estimation.

Description of Study Intervention: Fludrocortisone acetate (9-α-Fiuoro-11 β. 17 α, 21-trihydroxy-4-pregnene-3, 20 dione acetate) is a synthetic steroid possessing a potent mineralocorticoid effect and a high glucocorticoid activity. The physiologic effects of fludrocortisone acetate are similar to hydrocortisone but much more potent. FA has a glucocorticoid activity ten times higher than cortisol and a mineralocorticoid effect 250 times higher than cortisol. Addition of fluorine to C-9 of cortisol gives fludrocortisone a markedly increase in glucocorticoid, mineralocorticoid and anti-inflammatory potency. The drug will be administered via intravitreal injection.

Study Duration: 6-months' recruitment and 6-month follow-up period.

Participant Duration: Participants will be on the study for 6 months. Screening: up to 14 days Treatment: 1 day. Follow-up: 6 months.

Inclusion Criteria: Unless specified otherwise, ocular specific inclusion criteria apply to the study eye only. 1. Willing and able to give consent prior to any specific procedures being performed. 2. Male or Female. 3. Age ≥ 50 years. 4. Best corrected visual acuity (BCVA) of 24 letters or better using Early Treatment Diabetic Retinopathy Study (ETDRS) charts (20/320 Snellen equivalent). 5. Diagnosis of GA of the macula secondary to AMD in both eyes, confirmed within 14 days prior to dosing by the PI (Principal Investigator) using Fundus Autofluorescence (FAF) images, as well as the following criteria: a. Total GA area must be ≥ 1.9 and ≤ 17 mm2 (1 and 7 disc areas (DA) respectively), determined by screening images of FAF. b. If GA is multifocal, at least one focal lesions must be ≥ 1.25 mm2 (0.5 DA). c. GA can be completely visualized on the macula centered image. d. GA must be able to be photographed in its entirety. e. GA must be able to be measured separately from any areas of peripapillary atrophy. f. Presence of any pattern of hyperautofluorescence in the junctional zone of GA. Absence of hyperautofluorescence (i.e. pattern = none) is exclusionary. 6. Female subjects must be: a. Women of non-childbearing potential (WONCBP), or b. Women of childbearing potential (WOCBP) with a negative pregnancy test at screening and must agree to use protocol defined methods of contraception for the duration of the study. 7. Males with female partners of childbearing potential must agree to use protocol defined methods of contraception and agree to refrain from donating sperm for the duration of the study. 8. Willing and able to give informed consent. Note: If both eyes meet the inclusion criteria, the eye with the best visual acuity at the screening visit will be designated as the study eye. If both eyes have the same visual acuity, the right eye will be used as the study eye.

Exclusion Criteria: Unless specified otherwise, ocular specific exclusion criteria apply to the study eye only. 1. GA due to causes other than AMD such as Stargardt disease, cone rod dystrophy or toxic maculopathies like plaquenil maculopathy. 2. Spherical equivalent of the refractive error demonstrating > 6 dioptres of myopia or an axial length of >26 mm. 3. Evidence of exudative (wet) AMD including evidence of retinal pigment epithelium rips or evidence of neovascularization anywhere in the retina based on fluorescein angiogram as assessed by the PI in either eye within 12 months. 4. Retinal disease likely to confound visual performance or be affected by intraocular steroid. 5. Any ophthalmologic condition that reduces clarity of the media and that, in the opinion of the investigator interferes with ophthalmologic examination (e.g. advanced cataract or corneal abnormalities). 6. Any ophthalmologic condition that prevents adequate imaging of the retina judges by the PI. 7. Intraocular surgery (including lens replacement surgery) within 3 months prior to dosing. 8. Aphakia or absence of the posterior capsule. Previous violation of the posterior capsule is also excluded unless it occurred as a result of yttrium aluminum garnet (YAG) laser posterior capsulotomy in association with prior posterior chamber intraocular lens implantation and at least 60 days prior to Day 0. 9. Any ophthalmologic condition that may require surgery during the study period. 10. Glaucoma or family history of glaucoma. 11. Any contraindication of IVT injection including current ocular or periocular infection. 12. History of uveitis or endophthalmitis. 13. History of choroidal neovascularization (CNV) in either eye. 14. History of IVT injection within 12 months. 15. Participation in another interventional clinical study, or use of any experimental treatment for AMD or any other investigational new drug within 6 weeks or 5 half-lives of the active (whichever is longer) prior to the start of study treatment. Note: clinical trials solely involving observation, over-the-counter vitamins, supplements, or diets are not exclusionary. 16. Systemic conditions that are applicable to the use of fludrocortisone such as hypertension, and fungal infections. 17. Medical or psychiatric conditions that, in the opinion of the investigator, make consistent follow-up over the study period unlikely, or in general a poor medical risk because of other systemic diseases or active uncontrolled infections. 18. Any screening laboratory value (haematology, serum chemistry or urinalysis) that in the opinion of the investigator is clinically significant and not suitable for study participation. 19. Hypersensitivity to fluorescein.

Endpoints: The primary endpoints of the study are safety and tolerability of intravitreal (IVT) dose of Fludrocortisone acetate in subjects with geographic atrophy (GA) secondary to Age-Related Macular Degeneration (AMD), based on assessment of vital signs, clinical safety labs, and adverse events.

Primary Safety Endpoint: Number and severity of local and systemic treatment emergent events (TEAE).

Secondary Endpoints: The change in geographic atrophy (GA) lesion size from baseline to Month 6 as measured by FAF; Change in best corrected visual acuity (BCVA); Change in low luminance best corrected visual acuity (LL-BCVA); Relationship between GA lesion size changes and changes in BCVA; Increase in IOP.

Pharmacokinetic (PK) parameters: Exposure after single dose Fludrocortisone acetate IVT injections; Serum maximum observed concentration; Time to maximum measured concentration; Terminal elimination half-life.

Planned Interim Analysis: There is no formal interim analysis planned for this study. However, a data safety monitoring board (DSMB) for safety data review will be planned for this study.

Analysis Populations - the following analysis populations are planned: Safety Population: all enrolled participants IVT Fludrocortisone acetate will be included in the safety population; intention-to-treat (ITT) Population: all enrolled participants who received IVT Fludrocortisone acetate and had at least one efficacy measurement taken after dosing will be included in the ITT population.

Statistics Analyses- Safety Analysis: Statistical methods for the safety analyses will be primarily descriptive in nature. Listings and summaries for all safety data will be presented using the Safety Population. Descriptive statistics (mean, SD, median, minimum and maximum) will be calculated for summaries of continuous safety data and frequency counts and percentages (where appropriate) will be calculated for summaries of discrete/categorical safety data. Safety data, including vital signs, clinical safety labs and adverse events, will be summarised. Change from baseline will be included in summary tables for vital signs and laboratory parameters. All laboratory data will be included in the data listings and all test values outside the normal range will be flagged. Physical examinations will be listed for each participant. Adverse events (AEs) will be coded using the Medical Dictionary for Regulatory Activities (MedDRA), and data will be summarised by System Organ Class and preferred term.

Efficacy Analysis: exploratory analysis will be performed for the best-corrected visual acuity (BCVA) and will be scored with reference to the Early Treatment Diabetic Retinopathy Score (ETDRS letters). Geographic atrophy lesion size will be measured in mm2 by fundus autofluorescence imaging. Descriptive statistics (mean, SD, median, minimum and maximum) will be summarised for ETDRS letters and GA area in mm2 observed values and change from baseline at each post-injection visit. Exploratory analysis of ETDRS letter change and GA lesion size over time will be assessed by using a mixed model. The least squares mean of ETDRS and GA lesion size change from baseline and its 95% confidence interval at each visit will be estimated. Similar analyses will be conducted for intraocular pressures.

Categorical efficacy endpoints such as slit lamp biomicroscopy exam findings, dilated ophthalmoscopy exam findings, colour fundus photography and OCT findings will be summarised descriptively by frequency count and percentage (proportion) as appropriate.

Schedule of Activities: this is summarised in Table 4.

Formulation: The formulation is manufactured using aseptic processes. It is packaged in glass vials with coated rubber stoppers with plastic flip-off disks and are to be used for single-dose administration only. Part A: 10 mg of Fludrocortisone will be placed in a vial which will be sent off for Gamma Sterilization. Part B: Will be a vial containing the sterile diluent. These processes will be carried out by a trained Pharmacist and Sterile Facility technician in a Grade B Room under a Grade A hood. The investigational product will be produced by an accredited compounding pharmacy under GMP.

Storage and Handling: Fludrocortisone is formulated for intravitreal administration as a 10 mg powder for solution for injection.

Each vial contains 10 mg of fludrocortisone powder. The diluent consists of Isotonic Sterile Saline (Sodium Chloride solution 0.9%). The fludrocortisone powder for solution for intravitreal injection is reconstituted with 1.0ml or 0.5ml of diluent for injection depending on the concentration required. Following reconstitution, the concentration is suitable for delivering at a 1mg/0.1ml or 2mg/0.1ml dose in 0.1ml intravitreal injection volume.

The fludrocortisone 10mg powder for solution and diluent is for single use and must be stored at 2-8°C, protected from light.

Pharmaceutical Form: Fludrocortisone is formulated for intravitreal administration as a 10 mg powder for solution for injection.

Each vial contains 10 mg of fludrocortisone powder. The diluent consists of Isotonic Sterile Saline (Sodium Chloride solution 0.9%). The fludrocortisone powder for solution for intravitreal injection is reconstituted with 1.0ml or 0.5ml of diluent for injection depending on the concentration required. Following reconstitution, the concentration is suitable for delivering at a 1mg/0.1ml or 2mg/0.1ml dose in 0.1 ml intravitreal injection volume.

The fludrocortisone 10mg powder for solution and diluent is for single use and must be stored at 2-8°C, protected from light.

Therapeutic indications: Intravitreal Fludrocortisone is being developed for the treatment of age-related macular degeneration (AMD).

Dosage and Administration: Fludrocortisone may be provided as single-use stoppered glass vials containing sterile fludrocortisone 50mg.

The indicated IVT dose of fludrocortisone will be administered by one intravitreal injection of up to 0.1ml of 1mg/0.1ml and 2mg/0.1ml fludrocortisone.

Intravitreal toxicology: Intravitreal doses of Fludrocortisone acetate of 0 (vehicle control), 400µg, 1mg, 2mg, 4mg/eye were tested in a study of New Zealand albino rabbits.

All animals were examined before and after injection using the indirect ophthalmoscope and slit-lamp biomicroscopy. Electroretinography (ERG) was performed on all animals before intravitreal injection and two weeks after injection. The animals were re-examined at this time by indirect ophthalmoscopy and slit-lamp biomicroscopy and were euthanized. The eyes were enucleated and examined with light microscopy.

One eye in the 4 mg/0.1 ml fludrocortisone group exhibited significant decreases in ERG; one eye of this group had a vitreous hemorrhage. There was no significant decrease in ERG in the other groups. There were no signs of retinal toxicity on slit-lamp examination, indirect ophthalmoscopy, or by light microscopy in all eyes injected with 4 mg/0.1 ml or less of fludrocortisone. Intravitreal injections of fludrocortisone at 2 mg/0.1 ml appeared safe in albino rabbit eyes.

### ANIMAL STUDIES

An animal study was conducted to evaluate the safety and efficiency of Fludrocortisone acetate after intravitreal injection into the vitreous cavity [see Kivilcim, M., et al., Evaluation of the Retinal Toxicity of Fludrocortisone Acetate After Intravitreal Injection. Investigative Ophthalmology & Visual Science, 2006. 47(13): p. 4281-4281 ]. Surgeries were performed on the eyes of 25 New Zealand white rabbits. Fludrocortisone acetate was titrated using sterile BSS solution to the following concentrations: 4 mg/ 0.1 ml, 2 mg/ 0.1 ml, 1 mg/0.1 ml, and 400 µg/0.1 ml, which were injected intravitreal into one eye of twenty-five rabbit eyes. The control eyes received 0.1 ml of sterile BSS. All animals were examined before and after injection using indirect ophthalmoscope and slit-lamp biomicroscopy. Electroretinography (ERG) was performed on all animals prior to intravitreal injection and two weeks after injection. The animals were re-examined at this time by indirect ophthalmoscope and slit-lamp biomicroscopy and were euthanized. Their eyes were enucleated and examined with light microscopy.

Outcomes from these in vivo tests were positive for the application of intravitreal fludrocortisone acetate. In multiple measurements, up to 40 days of observations, the slit lamp biomicroscopy and indirect ophthalmoscopy did not show any evidence of significant inflammation in the anterior or posterior segment of the eye in either the control or treatment groups. IOP was normal across all groups.

Histological examination of retinal sections, under light microscopy, revealed that the integrity of the retinal layers appeared preserved with no evidence of toxicity or vacuolization except for one eye in the 4mg group. All aggregates of fludrocortisone acetate disappeared by 22 +/-8 days in the 2-mg group, and 33 +/-7 days in the 4-mg group.

In ERG testing, one eye injected with 4mg/0.1ml exhibited decreases in ERG output. All other eyes had a normal ERG. These findings represent promising results for intravitreal Fludrocortisone acetate that is clinically suitable for both short-term and long-term use.

Data from the light-damage model of dry AMD in mice showed FA delivered in Kenalog vehicle resulted in significantly less cell death, and significantly fewer macrophages that carry the IBA1 marker 7 days later. Histology shows that the animals treated with FA have a much thicker outer nuclear layer. Treated animals have a significantly more robust Electroretinogram A-wave (Photoreceptor function) and B-wave (inner retinal function).

### RATIONALE:

### RATIONALE FOR THIS STUDY:

Fludrocortisone acetate is available in tablet form (0.1 mg) from the PBS and so is approved for use in humans in Australia. In a study of 121 patients, excellent outcomes with fludrocortisone for anterior segment lesions were reported (see Gonzalez, C., Topical fludrocortisone (9-alpha fluorohydrocortisone) in ophthalmology. Am J Ophthalmol, 1960. 49: p. 619-22). This document cited 4 previous papers with similar experiences.

There is older pre-clinical data suggesting FA is effective. Among that is Fitzgerald et al. who found that FA was superior to TA in a study of phorbol-12-myristate-acetate (PMA)-stimulated monkey choroidal endothelial cells (CECs) in restoring quiescent morphology and reducing membrane permeability (Fitzgerald, M., et al., Mineralocorticoids restore quiescent morphology and reduce VEGF receptor expression in inflamed choroidal endothelial cells in vitro. Ophthalmic Res, 2009. 41(1): p. 44-52). Prof Jan Provis et al. have a large body of new preclinical data that indicates FA is superior to TA in a rat model of dry AMD. FA was superior in terms of preventing cell death, macrophage recruitment, and preserving a- and b-wave ERG amplitude.

Kivilcim cited above, examined intravitreal FA in 25 normal rabbits at 4 mg/0.1 ml, 2 mg/0.1 ml, 1 mg/0.1 ml, and 400 µg/0.1 ml. Two eyes at 4 mg/0.1 ml experienced intravitreal haemorrhage and reduced ERG. There were no other problems of reductions in ERG. The smaller volume of the rabbit eye would suggest 4 mg/0.1 ml would be safe in humans. TA is commonly given at 4 mg/0.1 ml so comparing the same doses for each steroid would be sensible.

In the current clinical study fludrocortisone acetate, corticosteroid inhibiting complement activation will be administered to patients with GA. The goal of the study is to assess the safety and tolerability of single dose IVT Fludrocortisone acetate. Results from this study will guide decisions to further develop intravitreal Fludrocortisone acetate for GA.

### DOSE SELECTION:

A single dose of 1mg/0.1mL or 2mg/0.1mL injection administered once will be tested in this study.

Intravitreal fludrocortisone acetate was well-tolerated in a panel of animal toxicology studies. The volume of the human vitreous is approximately 4 mL, which is approximately 2.7-fold larger than the mean vitreous volume of rabbits, 1.5mL. Based on the difference in vitreous volume between man and rabbit, the human equivalent dose was determined to be 67 mg/eye every 4 weeks. The dose (1- or 2mg/0.1 mL injection) of fludrocortisone acetate that will be evaluated in this clinical study is expected to result in drug concentrations approximately 1.3 fold lower than observed in rabbits.

### RISK/BENEFIT:

The safety monitoring practices employed by this protocol (e.g. complete ophthalmologic exam, IOP monitoring, OCT, vital signs, hematology, serum chemistry, and AE questioning) are adequate to protect the subjects' safety.

There are risks associated with the ophthalmic procedures required for participants in this study. However, these are all standard procedures that are widely performed in ophthalmology.

In the days following any IVT injection, patients are at risk of developing endophthalmitis. If the eye should become red, sensitive to light, painful, or develop a change in vision, the patient will be instructed to seek immediate care from an ophthalmologist. Other risks of IVT injection include traumatic cataract, retinal detachment and hemorrhage.

Transient increased IOP has also been identified as a risk following IVT injections. IOP will be carefully monitored in this study.

The approximately 150 mL of blood planned for collection from each subject over the 6 months of the study does not pose an undue risk in this patient population.

Based on data available to date, IVT administration of fludrocortisone acetate does not seem to present an unreasonable ophthalmic or systemic risk in animal models. Fludrocortisone acetate has been safely used systemically as mineralocorticoid replacement for severe orthostatic hypotension in Addison's disease and other salt- water disbalances.

However, as fludrocortisone acetate has not been used intravitreally in human participants there are potential unforeseen risks. To mitigate such risks, this study is being conducted in two parts, restricting treatment to a single individual together with extended assessment, prior to application in other participants. Numerous follow-up visits and numbers of testing procedures have also been instituted in the assessment schedule for all participants to ensure adverse events, or other safety issues that arise are identified and addressed in a timely manner.

There is a potential health benefit for trial participants from receipt of study drug. We propose to administer intravitreal fludrocortisone acetate to patients with GA. If efficacious, intravitreal fludrocortisone acetate is expected to alter the course of GA and slow its rate of progression.

### OBJECTIVES AND ENDPOINTS:

### STUDY OBJECTIVES:

The primary objectives of the study are to assess the safety and tolerability of IVT injections of Fludrocortisone acetate in subjects with GA associated with Age-Related Macular Degeneration in order to support further development into confirmatory Phase II studies.

### STUDY ENDPOINTS:

### PRIMARY SAFETY ENDPOINT:

To demonstrate safety and tolerability of IVT injections of Fludrocortisone acetate based upon the mean change in GA lesion size as measured by Fundus Autofluorescence (FAF). Number and severity of local and systemic treatment emergent adverse events.

### SECONDARY ENDPOINTS:

The change in square root geographic atrophy (GA) lesion size from baseline to Month 6 as measured by FAF. Change in best corrected visual acuity (BCVA) Change in low luminance best corrected visual acuity (LL-BCVA). Relationship between GA lesion size changes and changes in BCVA. Change in vital signs. Increase in IOP. Pharmacokinetic (PK) parameters. Exposure after single dose fludrocortisone acetate IVT injections. Serum maximum observed concentration.. Time to maximum measured concentration. Terminal elimination half-life.

### STUDY POPULATION:

The study population includes approximately 9 (n=9) subjects to be enrolled at approximately one (1) site. To participate in the study, subjects must be diagnosed with GA of the macula associated with AMD in both eyes. If both eyes meet the criteria and qualify for the study, the eye with the best visual acuity at the screening visit will be designated as the study eye. If both eyes have the same visual acuity, the right eye will be used as the study eye.

The complete inclusion and exclusion criteria are presented above.

WOMEN OF CHILDBEARING POTENTIAL (WOCBP):
WOCBP are defined as pre-menopausal women physiologically capable of becoming pregnant.
WOMEN OF NON-CHILDBEARI NG POTENTIAL:
WONCBP are defined as women meeting any of the following criteria: Older than 45 years with amenorrhea for > 2 years or older than 60 years with amenorrhea for > 1 year, both confirmed by FSH and LH levels; Has undergone hysterectomy; Has undergone bilateral oophorectomy; and Has undergone bilateral salpingectomy.

### APPROVED METHODS OF CONTRACEPTION:

Approved methods of contraception include: oral contraceptives, intrauterine device, medically acceptable barrier methods (i.e. condom), implantable or injectable contraceptives or removable birth control device. Subjects practicing abstinence and coitus interruptus (pull out method) must agree to use an approved method of contraception during the study

### TREATMENT OF SUBJECTS:

### ALLOCATION OF TREATMENT:

Each subject will be assigned a unique screening number before screening. Subjects who complete the study screening assessments and meet all the eligibility criteria will be scheduled to enter the study and will receive treatment with intravitreal fludrocortisone acetate on Day 0.

### TREATMENTS ADMINISTERED:

### DOSE LEVELS AND STUDY ARMS:

A single dose of 1- and 2 mg Fludrocortisone acetate/0.1mL will be tested in this study.

Subjects will receive 1 IVT injection as outlined in Table 5.

### DRUG SUPPLIES:

### IDENTITY OF INVESTIGATIONAL PRODUCT

Fludrocortisone acetate is formulated for intravitreal administration as a 20mg powder for solution for injection.

Each vial contains 50mg of fludrocortisone powder. The diluent consists of Carboxymethyl cellulose (0.6%), Polysorbate 80 (0.02%) and Sodium Chloride solution (0.9%) quantity sufficient to 100%. The fludrocortisone powder for solution for intravitreal injection is reconstituted with 1.0ml and 0.5ml diluent for injection dependent upon concentration required. Following reconstitution, the concentration is suitable for delivering at a 1mg/0.1ml or 2mg/0.1ml dose in 0.1 ml intravitreal injection volume.

The fludrocortisone acetate 50mg powder for solution and diluent is for single use and must be stored at 2 to 8°C, protected from light.

### ACCOUNTABILITY:

IVT Fludrocortisone acetate drug product will be provided to a designee at the study site and must be stored in a pharmacy or otherwise locked and secured, at temperatures between 2°C and 8°C in a refrigerated area with limited, controlled access and temperature monitoring; do not freeze. IVT Fludrocortisone acetate drug should be protected from light by storing in the carton provided. The drug product supply is accessible only to those individuals authorized by the PI. The Sponsor will supply sufficient quantities of fludrocortisone acetate drug product to allow completion of this study.

Designated study staff will provide the study treatments to the subjects in accordance with their assigned subject numbers and the randomization schedule. During the study, the receipt of the drugs supplied at the clinical site and of study treatment dispensation for each subject will be documented in drug accountability records. These drug accountability records are to be kept separate from the patient medical records and other source documents.

All used vials should be retained by the clinical site until drug accountability monitoring is performed and then returned to the Sponsor or designee, or destroyed per Sponsor instructions.

At the conclusion of the study, any unused investigational product will be retained by the clinical site, returned to the Sponsor or designee, or destroyed per Sponsor instructions, and this will be documented in the drug accountability records.

### INTRAVITREAL FLUDROCORTISONE ACETATE ADMINISTRATION:

Subjects receiving active treatment will be administered a 1mg/0.1ml or 2mg/0.1mL IVT injection of Fludrocortisone acetate using a 27G thin wall needle, at the discretion of the PI.

Clinic staff involved in the injection tray assembly, anaesthetic preparation, and study drug preparation and administration will follow appropriate aseptic techniques to minimize the risk of potential adverse events associated with IVT injections (e.g. endophthalmitis).

The investigation drug will be presented in a kit containing 1 x 1 ml syringe with 27g 12 mm needle, 1 x 5 ml vial containing sterile fludrocortisone 10mg, 1 x Vial containing 2 ml Diluent for reconstitution and alcohol swab to swab top of vials before puncturing bungs.

Preparation of the solution: The injecting doctor will withdraw 1.0ml or 0.5ml of diluent and reconstitute the powder. The doctor will then draw up 0.1 ml of the fluid ready for injection.

To minimize IOP elevation after IVT injection of fludrocortisone acetate, decompression of the eye must be performed before all fludrocortisone acetate injections. This is done by applying moderate pressure to the globe with cotton swabs for 30-60 seconds during aesthetic preparation.

In addition to the procedures outlined in this protocol, adherence to specific institutional policies associated with IVT injections will be observed.

### CONCOMITANT THERAPIES:

Any concomitant medications a participant is receiving at the start of the study or that are given for any reason during the study (except for routine medications given for ocular procedures required by the protocol, such as topical aesthetic) must be recorded in the source document and CRF including start and stop date and time, dose, route, and indication. In addition, all ocular and non-ocular procedures such as surgical procedures (excluding study treatment procedures) must also be recorded in the source document including start and stop dates. Surgical anaesthetics, paramedical or alternative therapies (e.g. acupuncture, massage) should also be recorded in the source documents and CRF. Metoclopramide or other agents to prevent nausea induced by fluorescein injection may be administered at the discretion of the PI.

### ENDOPHTHALMITIS TREATMENT:

The decision to treat a participant for endophthalmitis or suspected endophthalmitis will be guided by the clinical judgment of the PI. The treatment method (pars plana vitrectomy vs. vitreous tap) and choice of antimicrobial agents are also at the discretion of the PI and should follow current standard practice patterns. The decision to use IVT steroids (e.g. dexamethasone) for the treatment of endophthalmitis is also at the discretion of the PI.

### STUDY PROCEDURES:

### STUDY DESIGN:

This is a Phase Ib study to assess the safety and tolerability of a single dose of IVT injection of fludrocortisone acetate in subjects with GA associated with Age-Related Macular Degeneration.

Patients diagnosed with GA associated with age-related macular degeneration in the study eye and who meet all inclusion/exclusion criteria will be included in the study.

The study is planned to enrol an initial cohort of 3 patients in a dose of 1mg/0.1mL. A total of 9 participants can be included to assess dose escalation based on 3+3 algorithm.

Patients should be screened up to 14 days before receiving Fludrocortisone acetate. Upon entry into the study, patients will be assigned a subject screening number. Subjects who meet all inclusion and exclusion criteria and are confirmed as eligible by the CRC will return to the clinic for the administration of single dose IVT Fludrocortisone acetate (Day 0) as outlined below.

All subjects will return to the clinical site on Day 1 and Day 7 to assess acute safety after the injection. After that, all subjects will return for another 6 follow-up visits and 6 months after the injection. See Study Outline below.

Safety will be assessed throughout the study; serial blood samples and urine samples will be collected. Blood samples will also be collected for the PK assessment of fludrocortisone acetate.

The planned length of participation in the study for each subject is approximately 6 months (from Day 0 - through completion of the Month 6 (Day 150) follow-up procedures).

The study is planned to take place over approximately 12 months (from screening of the first subject through completion of the last subject's exit visit).

### SUBJECT ENROLL MENT:

It is the responsibility of the investigator to ensure that subjects are eligible to participate in the study prior to enrolment and throughout the study. Documentation of the personally signed and dated informed consent of each subject, using the study-specific ICF, is required before initiating the Screening process. After written informed consent has been obtained and eligibility to participate established, investigative site personnel will obtain the subject's identification number. Only eligible subjects will be allocated to the open label FA 1 mg/0.1mL or 2mg/0.1 mL.

Enrolment will occur in two parts in order in order to minimise the likelihood that subjects will be exposed to risks. During part 1 & 2, subjects will be screened one by one as only one patient will initially be enrolled during part 1 of the trial. The first enrolled subject will participate in a screening period of up to 14 days and a follow-up period of 28 days, to detect an IOP response.

After the first subject has completed the follow-up of 28 days after FA 1mg/0.1 mL injection, The subject's safety data will be reviewed the safety data of this will be reviewed by an independent safety review committee to determine whether to commence enrolment of additional 2 patients in the first cohort of 1mg/0.1mL dose of FA. Enrolment will be stopped if >2 patients experience limiting-toxicity adverse event related to the study drug. If no more than two adverse events considered to be related to the study drug occur with limiting toxicity, the second cohort will be recruited. Dose-limiting toxicity is defined by intraocular inflammation, elevated IOP, reduced vision (loss of ≥15 letters), or haemorrhage within 28 days after injection.

Part 2 will take in place if the 3 subjects have tolerated well the dose. Part 2 initially involves a single subject treated with 2mg/0.1mL FA to assess safety and tolerability. This subject will be followed for up to 28 days and reviewed by an independent safety review committee prior to the recruitment of a further 5 subjects treated with 2mg/0.1mL FA totalling in 6 subjects in the second cohort.

Meeting minutes will be generated at each meeting and included in the sponsor's study files. Formal reports will not be prepared prior to or following these meetings. As general guidance, a subject will be considered to have tolerated a dose if the subject experiences no clinically significant drug-related adverse event or laboratory abnormality. Conversely, a subject will not be considered to have tolerated the dose if he experiences a clinically significant drug-related adverse event or laboratory abnormality during the study drug administration or post-administration follow-up period

Safety to cataract will be reviewed among all participants at the 150 day review.

It is understood that safety is a medical judgment that cannot be prospectively defined in detail. Subjects will be closely monitored with clinical observations and safety laboratory testing.

### STUDY VISIT SCHEDULE

Below is a condensed description of the study visits and the procedures and examinations that will be performed. Please refer to the Schedule of Activities (SoA) table for a detailed schedule of procedures/assessments for the Monthly visit schedules. Additional safety assessments not listed herein may be performed if considered necessary at the discretion of the PI.

### SCREENING - WITHIN 14 DAYS PRIOR TO TREATMENT

Visit 1 - All Subjects: All ophthalmic procedures (including imaging) are to be performed on both eyes:
1. Before any study specific procedures are performed, explain the purpose and nature of the study, and have the patient read, sign, and date the Institutional Review Board/Independent Ethics Committee (IRB/IEC) - approved Informed Consent Form (ICF). Have the individual obtaining consent from the patient and a witness, if applicable, sign and date the ICF. 2. Obtain a screening number for the subject. 3. Obtain information on demographics, medical/ocular history, and concomitant medications used 90 days prior to enrolment. Include vitamins, and all over-the-counter as well as prescription medications. 4. Screen the patient for inclusion/exclusion criteria. 5. Collect blood (including blood for HCG/FSH/LH, if applicable) and urine for laboratory analysis and forward the samples to the central laboratory. 6. Collect vital signs. 7. Perform BCVA. 8. Perform LL-BCVA. 9. Perform a complete ophthalmic exam including slit-lamp exam of the cornea, iris, anterior chamber, lens (LOCS III if any opacity on the lens noted) and aqueous reaction (cells and flare), dilated fundus exam of the vitreous and retina and IOP measurement. 10. Perform SD-OCT imaging for determination of eligibility by the PI. 11. Perform FAF imaging for determination of eligibility by the PI. 12. Perform NIFR imaging. 13. Perform DCFP for determination of eligibility by the PI. 14. Perform FA for determination of eligibility by the PI.

Visit 2 (Baseline) - All subjects: Unless specified, all ophthalmic procedures (including imaging) are to be performed on the Study eye only. 1. Verify that all inclusion/exclusion criteria are met, including the determination of eligibility by the PI. 2. Obtain information on any changes in medical health and/or the use of concomitant medications. 3. Collect vital signs pre- and post-dose. Vital signs will be measured within 1 hour prior to dosing for the pre-dose time point. Post-dose vital signs readings will be performed within 30 minutes after dosing. 4. Perform BCVA. 5. Perform LL-BCVA. 6. Perform a complete ophthalmic exam including slit-lamp exam of the cornea, iris, anterior chamber, lens (LOCS III if any opacity on the lens noted) and aqueous reaction (cells and flare), dilated fundus exam of the vitreous and retina and IOP measurement. 7. Perform the IVT injection of fludrocortisone acetate. 8. Monitor the study eye within 15 minutes' post injection. 9. Monitor for adverse events.

Visit 3 (Day 1): All Subjects. Post-initial treatment examination:
Unless specified, all ophthalmic procedures (including imaging) are to be performed on the study eye only. 1. Obtain information on any changes in medical health and/or the use of concomitant medications. 2. Collect vital signs. 3. Perform BCVA. 4. Perform LL-BCVA. 5. Perform a complete ophthalmic exam including slit-lamp exam of the cornea, iris, anterior chamber, lens (LOCS III if any opacity on the lens noted) and aqueous reaction (cells and flare), dilated fundus exam of the vitreous and retina and IOP measurement.

### Monitor for adverse events:

Follow-up Visits - Day 7, 14, 28, 60, 90: Unless specified, all ophthalmic procedures (including imaging) are to be performed on the study eye only. The following procedures will be performed at all follow-up visits: 1. Obtain information on any changes in medical health and/or the use of concomitant medications. 2. Collect vital signs 3. Collect blood for PK analysis (days 7, 28 and 90 only). 4. Perform a complete ophthalmic exam including slit-lamp exam of the cornea, iris, anterior chamber, lens (LOCS III if any opacity on the lens noted) and aqueous reaction (cells and flare), dilated fundus exam of the vitreous and retina and IOP measurement. 5. Monitor for adverse events Termination Visit (or Early Termination) - Day 150: All ophthalmic procedures are to be performed on BOTH EYES. 1. Obtain information on any changes in medical health and/or the use of concomitant medications. 2. Collect blood and urine for laboratory analysis and forward the samples to the central laboratory. 3. Collect blood for PK analysis. 4. Collect vital signs. 5. Perform urine pregnancy test. -WOCBP only.

6. Perform BCVA. 7. Perform a complete ophthalmic exam including slit-lamp exam of the cornea, iris, anterior chamber, lens (LOCS III if any opacity on the lens noted) and aqueous reaction (cells and flare), dilated fundus exam of the vitreous and retina and IOP measurement. 7. Perform SD-OCT imaging. 8. Perform FAF imaging. 9. Perform NIFR imaging. 10. Perform DCFP. 11. Perform FA imaging. 12. Monitor for adverse events

### Unscheduled Visit:

If a subject return to the clinical site before their next scheduled visit for an assessment of an adverse event or at the request of the PI, all assessments completed at the Unscheduled Visit should be documented in the patient source record and in the eCRF.

### LOST TO FOLLOW-UP:

A participant will be considered lost to follow-up if he or she fails to return for ≥1 scheduled visits and is unable to be contacted by the study site staff.

The following actions must be taken if a participant fails to return to the clinic for a required study visit:
- The site will attempt to contact the participant and reschedule the missed visit within one week and counsel the participant on the importance of maintaining the assigned visit schedule and ascertain if the participant wishes to and/or should continue in the study.
- Before a participant is deemed lost to follow-up, the investigator or designee will make every effort to regain contact with the participant (where possible, 3 telephone calls and, if necessary, a certified letter to the participant's last known mailing address or local equivalent methods). These contact attempts should be documented in the participant's medical record or study file.
- Should the participant continue to be unreachable, he or she will be considered to have withdrawn from the study with a primary reason of lost to follow-up.]

### STUDY ASSESSME NTS AND PROCEDURES

The following evaluations will be performed during the study outlined in the Schedule of Activities.

### INFORMED CONSENT PROCEDURES

The Principal Investigator(s) at each site will ensure that the subject is given full and adequate oral and written information about the nature, purpose, and possible risk and benefit of the study. Subjects must also be notified that they are free to discontinue from the study at any time. The subject should be given the opportunity to ask questions and allowed time to consider the information provided.

The subject's signed and dated informed consent must be obtained before conducting any study procedures.

The Principal Investigator(s) must maintain the original, signed Informed Consent Form. A copy of the signed Informed Consent Form must be given to the subject.

### VITAL SIGNS

On injection visit, vital signs will be measured within 1 hour prior to dosing and within 30 minutes after dosing.

Vital signs will be measured before venipuncture. Vital signs include blood pressure (BP) and pulse measurements. After the patient has been sitting for 3 minutes, with back supported and both feet placed on the floor, systolic and diastolic BP will be measured using an automated validated device, with an appropriately sized cuff. In case the cuff sizes available are not large enough for the patient's arm circumference, a sphygmomanometer with an appropriately sized cuff may be used. If vital signs are out-of-range at screening/eligibility, the Investigator may obtain two additional readings, so that a total of up to three consecutive assessments are made, with the patient seated quietly for approximately five minutes preceding each repeat assessment. At least the last reading must be within the ranges provided above in order for the patient to qualify. All of the above tests will be performed after resting for 3 minutes at all visits.

Height in centimetres (cm) and body weight (to the nearest 0.1 kilogram [kg] in indoor clothing, but without shoes) will be measured at Visit 1 (Screening). Body mass index (BMI) will be calculated using the following formula: BMI = Body weight (kg) / [Height (m)]2.

### LABORATORY ANALYSIS OF BLOOD AND URINE

Collection of blood and urine will occur at the study site and the samples will be shipped to a central laboratory for analysis.

The following clinical labs will be performed: Hematology: Hemoglobin; Hematocrit; Red blood cell (RBC) count; Platelet count; white blood cell (WBC) count with differential; Chemistry: Blood urea nitrogen (BUN); Creatinine; Bilirubin (total, direct and indirect); Albumin; Alkaline phosphatase (ALP); Aspartate aminotransferase (AST); Alanine aminotransferase (ALT); Creatine kinase; Glucose; Electrolytes (sodium, potassium, chloride, bicarbonate); Urinalysis: pH; Specific gravity; Protein; Glucose; Ketones; Bilirubin; Blood; Nitrite; Urobilinogen; Leukocyte esterase; Other: Human chorionic gonadotropin (HCG) a; Follicle-stimulating hormone (FSH) b; Luteinizing hormone (LH) b.

The Investigator must review the results of the Screening Visit clinical laboratory tests (including recheck results) and confirm that these results do not show evidence of any medical condition that would make study participation inappropriate. The Investigator should also assess any changes from baseline at the follow up visits and the Exit Visit.

Notes: a. Serum Pregnancy Test (i.e. HCG) will be performed for females of child bearing potential at screening only.b. FSH and LH will be performed for postmenopausal females at screening only.

### URINE PREGNANCY TEST

Urine pregnancy test will be performed in WOCBP only as outlined in the Study Flow Chart.

### BEST-CORRECTED VISUA L ACUITY

Best-corrected visual acuity (including LL-BCVA) testing, performed by a certified VA examiner, should precede any examination requiring administration of eye drops to dilate the eye or any examination requiring contact with the eye. ETDRS best-corrected visual acuity (BCVA) will be obtained in each eye separately at screening (Visit 1). This assessment is to be performed prior to pupil dilation. The number of letters read correctly (for each eye) will be recorded in the appropriate study document. For the remainder of study visits (Visits 2-8), BCVA will only be obtained in the study eye.

### COMPLETE OPHTHALMIC EXAM

The complete ophthalmic exam will consist of the following: External examination of the eye and adnexa; Routine screening for eyelids/pupil responsiveness (including ptosis, abnormal pupil shape, unequal pupils, abnormal reaction to light and afferent pupillary defect); Slit-lamp examination [cornea, anterior chamber, iris, lens, aqueous reaction (cells and flare). If an abnormal lens finding is noted during the slit-lamp examination, at any visit, then the finding should be further characterized with LOCS III. All subsequent visits for that subject should include LOCS III. A complete description of LOCS III standardized procedures and grading scales is outlined in the MOP; Dilated fundus exam including evaluation of retina and vitreous (i.e. posterior segment abnormalities, retinal hemorrhage/detachment, and vitreal hemorrhage density and vitreous cells); Vitreal hemorrhage density and vitreous cells grading scales; Intraocular pressure (IOP) will be measured in both eyes at Visit 1 as per the study site's regular practice and recorded in the appropriate study document. For the remainder of study visits (Visits 2-9), IOP will only be obtained in the study eye.

### OCULAR IMAGING

The following ocular images will be obtained as outlined in the visit schedule above, also see SOA: Digital Color Fundus Photograph; Fluorescein angiography; Spectral Domain Optical coherence tomography; Fundus Autofluorescence; Infrared reflectance imaging. Only done at selected clinical sites with Heidelberg Spectralis^{®} system.

### POST-INJECTION ASSESSMENT

The study eye will be assessed before and after injection to ensure that the injection procedure and/or the study medication have not endangered the health of the eye. The initial post-injection assessment should be done within 15 minutes post-injection and include a gross assessment of vision (light perception) and monitoring IOP. If subject passes gross vision test and IOP is < 30mmHg, the subject may leave the site. If subject fails gross vision test and/or IOP is > 30 mmHg, assessments will continue every approximately 30 minutes until the subject passes gross vision test and IOP is 30 mmHg.

Any subject who develops a significant and sustained raise in IOP (> 30 mmHg) or a non-adequately perfused central retinal artery (CRA) after injection, should be monitored according to the PI's clinical judgment and may undergo additional procedures and measurements of IOP beyond those specified in the protocol as well as IOP lowering procedures. If any concern or immediate toxicity is noted, the subject will remain at the site and will be treated according to the PI's clinical judgment.

### BLOOD VOLUME FOR STUDY ASSESSMENTS

Blood volume during study (up to Day 150), see Table 6.

### ADVERSE EVENTS AND SERIOUS ADVERSE EVENTS

All adverse events (AEs) (as defined above), either observed by the PI or one of their medical collaborators, or reported by the participant spontaneously, or in response to direct questioning, will be reported. All adverse events (ocular, non-ocular, serious, non-serious, volunteered, and elicited) must be documented in study records.

### DEFINITION OF ADVERSE EVENTS (AE)

An adverse event is any untoward medical occurrence in a subject who receives a pharmaceutical product. The occurrence does not necessarily have to have a causal relationship with the treatment. Therefore, an AE can be any unfavorable and unintended sign, symptom, or disease temporally associated with the use of a drug, whether or not considered related to the drug.

Note: For purposes of this study, abnormal laboratory values will not be considered adverse events unless deemed clinically significant by the Investigator. All abnormal laboratory values will be recorded in the database and appropriate analyses presented in the final study report.

### DEFINITION OF SERIOUS ADVERSE EVENTS (SE)

A serious adverse event (SAE) is defined as any untoward medical occurrence that at any dose: Results in death; is life-threatening: this means that the subject was at risk of death at the time of the event; it does not mean that the event might have caused death had it occurred in a more severe form; Required hospitalization or prolongation of existing hospitalization; results in persistent or significant incapacity or substantial disruption of the ability to conduct normal life functions; or is a congenital anomaly or birth defect.

Important medical events that may not result in death, be life-threatening, or require hospitalization may be considered serious when, based upon appropriate medical judgment, they may jeopardize the patient or subject and may require medical or surgical intervention to prevent one of the outcomes listed in the above definition.

Medical and scientific judgment should be exercised in deciding if an AE is serious and if expedited reporting is appropriate.

### ADVERSE EVENTS OF SP ECIAL INTEREST

An adverse event of special interest is one of scientific and medical concern specific to the Sponsor's product or program where ongoing monitoring and rapid communication by the Investigator to the Sponsor may be appropriate. These adverse events may be serious or non-serious. Applicable adverse events may require further investigation in order to characterize and understand, and depending upon the nature of the event, rapid communication by the trial Sponsor to other parties may also be required. These adverse events of special interest must be reported using the same mechanism and time frame (i.e. within one working day of the Investigator's or delegate's knowledge of the event) as described for serious adverse events. The adverse events of special interest include the following: Endophthalmitis; 4+ ocular inflammation; 2-3+ ocular inflammation that fails to decrease to 1+ or less within 30 days of the onset of the event; Sustained (> 5 minutes) loss of light perception after FA injection; Sustained elevation of IOP (30 mmHg) at/past 90 minutes' post-injection; Any elevation of IOP requiring surgical intervention (i.e. paracentesis); new vitreous hemorrhage of > 2+ severity that does not resolve within 14 days of the onset of the even; cataract progression.

If an adverse event of special interest occurs in a study subject, the study subject will be followed for resolution of the adverse event. A decision will be made by the Sponsor concerning further exposure to the study treatment and further participation in the study.

### ADVERSE EVENT ASSESSMENT AND RECORDING

The Investigator will probe, via discussion with the subject, for the occurrence of AEs during each subject visit and record the information in the site's source documents. For each AE, the PI should note the start and resolution dates, the severity, whether it meets the definition of an SAE, the relationship of the event to the study drug, the action taken regarding study drug, and the outcome of the event. Data should be transcribed from the source documents to the CRF as per the CRF instructions.

When reporting an adverse event, the event description should use the best matching terminology describing the event as found in the "Common Terminology Criteria for Adverse Events" (CTCAE, v 4.03). If an available CTCAE term fits the event well, no additional descriptors may be needed.

However, the Investigator should add any necessary descriptions in order to clarify the event or to place it in an appropriate context. If an appropriate term matching the adverse event cannot be found in the CTCAE and you do not know the preferred MedDRA term, the adverse event description should include a diagnosis, sign or symptom with additional information to facilitate subsequent categorization into MedDRA coding terms

### INTENSITY

The PI must grade the severity of all reported adverse events into one of five categories: Grade 1 (Mild), Grade 2 (Moderate), Grade 3 (Severe), Grade 4 (Life-Threatening) or Grade 5 (Death related to AE). The standardized CTCAE severity grading scales for the specific type of adverse event reported must be used when a matching CTCAE term is available. If no reference to a standard grading scale applies or is immediately available, use the following guideline:
GRADE 1 -MILD: Persistence of any otherwise insignificant medical occurrence beyond 72 hours or any transient (< 72 hours) AE considered by the PI to be related to the study drug. No or minimal medical therapy or intervention required, hospitalization not necessary, no or little limitation in normal activities; non-prescription or single-use prescription therapy may be employed to relieve symptoms. Mild adverse events may be listed as expected consequences of the therapy for any given protocol, and standard supportive measures for such an expected event do not necessarily elevate the event to a higher grade.
GRADE 2- MODERATE: Mild to moderate limitation in activity, some assistance may be needed; possibly none but usually minimal intervention/therapy required, hospitalization possible.
GRADE 3- SEVERE: Marked limitation in activity, some assistance usually required; medical intervention/therapy required; hospitalization possible or likely. [Specifically, for ocular adverse events in this vision related study, an immediately sight-threatening condition (e.g., impending corneal perforation, retinal detachment) may be categorized as Grade 3 if it would lead to total blindness in the affected eye(s).]
GRADE 4- LIFE THREATENING: Extreme limitation in activity, significant and immediate assistance required; significant medical/therapy intervention required to prevent loss of life; hospitalization, emergency treatment or hospice care probable. This grade is used when the participant was, in the view of the PI, at substantial risk of dying at the time of the adverse event or it was suspected that use or continued use of the test article would have resulted in the participant's death. (This does not include a reaction that, had it occurred in a more serious form, might have caused death. For example, drug-induced hepatitis that resolved without evidence of hepatic failure would not be considered life-threatening even though drug-induced hepatitis can be fatal.)
GRADE 5- DEATH: Death related to AE.

### CAUSALITY:

The PI (or an authorized study physician) must submit an attribution for causality of the reported adverse event to the test article or procedure.

The attribution should take into account both the temporal association and any known physical, physiological or toxicological information regarding the test article that could reasonably infer causality. Causality should only be considered for the experimental test article and not for any standard study examination or diagnostic procedures. The four attribution categories are: Unrelated - Does not follow a reasonable temporal sequence from the administration of study drug. The event or laboratory test abnormality is clearly due to extraneous causes (disease, other drugs, environment, etc.) Unlikely related - Does not follow a known pattern of response to study drug. Does not follow a reasonable temporal sequence from the administration of study drug. Disease or other drugs provides plausible explanation.

It does not reappear or worsen when study drug is re-administered Possibly related - Follows a known pattern of response to study drug. Time sequence from administration of the study drug is reasonable. Could also be explained by disease or other drugs. Probably related - Follows a known pattern of response to study drug. Time sequence from administration of the study drug is reasonable. Response to withdrawal clinically reasonable. Cannot be reasonably explained by the known characteristics of the participant's clinical state, environmental factors, or other therapies administered to the subject.

### SERIOUS ADVERSE EVENT REPORTING

All SAEs (defined herein), whether judged related or not to study medication, will be reported to the Sponsor (or designated Medical Monitor) by telephone, e-mail or facsimile within 24 hours of the Investigator becoming aware of such SAEs. The contact details can be found of the serious adverse event form.

The initial SAE Report should include, at a minimum, the following information: Protocol number; Site number; Subject screening number, initials, gender, and date of birth; Name of PI and investigator site address; Details of SAE; Criterion for classification as "serious"; Date of SAE onset.

Follow-up SAE reports should be submitted as further information becomes available, and the final SAE Report should include information on the SAE intensity, outcome, and relationship to study drug; dates of study drug administration, concomitant medications, and any other relevant information. The PI should also provide clear copies of supporting documents as necessary (e.g. hospital discharge summary, laboratory reports, autopsy reports, etc.), with the subject's personal identifiers removed. All SAEs will be followed until the acute event has resolved, even if the subject discontinues study participation prior to the resolution. The Investigator must report SAEs occurring at his/her site to the IRB/IEC as required.

### EXPECTED ADVERSE EVENTS

### EXPECTED AE RELATED TO THE TEST ARTICLE:

No ocular or systemic AE related to the investigational drug are expected at the doses proposed in this protocol.

### EXPECTED AE RELATED TO THE IVT INJECTION PROCEDURE:

Mild discomfort related to the injection procedure (including use of an eyelid speculum, anaesthetic drops, mydriatic drops, antibiotic drops, povidone-iodine drops or flush and subconjunctival injection of anaesthetic, as well as the actual insertion of the IVT needle) are expected. These procedure-related adverse events include but are not limited to: redness, mild eye pain, eye irritation, visual disturbance, abnormal sensation in the eye, etc. and will be graded as indicated herein

### DISEASE PROGRESSION

A condition considered by the PI as unequivocal AMD disease progression in the study eye or fellow eye should be identified as such in the participant's source documents and should not be recorded as an adverse event in the CRF, such as lesion growth, lesion bleeding, lesion that exudes fluid, an RPE tear, and extensive deposition of lipid. All other conditions should be recorded as an adverse event. The unequivocal nature of the disease progression must be indicated in the source documents. Normal progression or worsening of the medical condition under study (e.g. vision loss due to the progression of AMD), by itself, does not necessarily constitute an adverse event unless the change can be reasonably attributed to an action of the test article and not only to its lack of efficacy.

### WITHDRAWAL

Participants may choose to withdraw from this study for any reason at any time without penalty or prohibition from enrolling in other clinical protocols.

Participant wishing to withdraw from the study completely will be offered an early termination visit.

This early termination visit will include the examinations outlined herein.

### PREGNANCY IN THE CLINICAL TRIAL

WOCBP are not excluded from the study as long as adequate birth control methods are being utilized. Prior to enrolment in the clinical trial, WOCBP must be advised of the importance of avoiding pregnancy during the trial and the potential risks associated with an unintentional pregnancy. WOCBP and males with partners who are WOCBP will be instructed to practice an acceptable method of birth control (as defined above) for the duration of the study. Male subjects will be counselled to avoid donating sperm after dosing on Day 1 until the final Exit visit.

During the trial, female subjects are to be instructed to contact the Investigator immediately if they suspect they might be pregnant. The study Sponsor must be contacted immediately and a decision will be made regarding continuation of the pregnant woman in the study based upon the circumstances surrounding the pregnancy. Pregnancy is not reportable as an adverse event; however, complications may be reportable. If a female subject or partner of a male subject becomes pregnant during the study, the PI should report the pregnancy to the Medical Monitor within 24 hours of being notified. The Investigator should follow the pregnancy until completion. At the completion of the pregnancy, the Investigator will document and report the outcome. If the outcome of the pregnancy meets the criteria for classification as an SAE (i.e. postpartum complication, stillbirth, neonatal death, or congenital anomaly) the Investigator should follow the procedures for reporting an SAE.

### STATISTICAL CONSIDERATIONS

Descriptive summaries will include mean, standard deviation, median, and range for continuous variables and counts and percentages for categorical variables.

### POPULATION FOR ANALYSIS

Safety Analysis: All subjects who received at least one dose of treatment will be included in the evaluation of safety of FA.

Efficacy Endpoint(s): The efficacy analysis will be based on an intention-to-treat population (ITT), which is defined as all subjects who received the single dose of treatment and have at least one visit at or after month 2. Month 2 is the first visit on treatment at which lesion area is measured. Per protocol (PP) efficacy analyses will include all randomized subjects who return for Day 150 of follow up.

Safety is the main analysis population for safety endpoints, and ITT is the main analysis population for efficacy endpoints. The assignment of participants to each analysis population will be based on the review of data after the completion of all data collection, monitoring by the clinical research associate and first round of query resolution by data management and prior to database lock.

### DEMOGRAPHIC AND BASELINE CHARACTERISTICS

Participant demographic and baseline variables (age, sex, ethnicity, race, height, weight, and BMI) will be summarised with descriptive statistics. Sex, ethnicity, and race will be summarised with frequency counts and percentages. Baseline ocular assessments will be summarised descriptively as well.

Pregnancy test results, concomitant medication and medical history data for each participant will be presented in data listings. Concomitant medications will be summarised descriptively by using frequency counts and percentages.

### ANALYSIS OF PRIMARY SAFETY ENDPOINTS

No formal inferential statistics will be performed on safety assessments. Statistical methods for the safety analyses will be primarily descriptive in nature. The Fludrocortisone acetate 1mg/0.1mL and 2mg/0.1 mL injection will be considered as safe and tolerable based on the number of subjects presenting severe AEs related to the study drug. It is understood that safety is a medical judgment that cannot be prospectively defined in detail. However, as general guidance, a subject will be considered to have tolerated a dose if the subject experiences no clinically significant drug-related adverse event or laboratory abnormality. Conversely, a subject will not be considered to have tolerated the dose if he experiences a clinically significant drug- related adverse event or laboratory abnormality during the study drug administration or post-administration follow-up period.

Listings and summaries for all safety data will be presented using the Safety Population. Descriptive statistics (mean, SD, median, minimum and maximum) will be calculated for summaries of continuous safety data and frequency counts and percentages (where appropriate) will be calculated for summaries of discrete/categorical safety data.

Adverse events (AEs) will be coded using the Medical Dictionary for Regulatory Activities (MedDRA), and data will be summarised by System, Organ, Class and preferred term. The number and percent of participants reporting each AE will be summarised descriptively (n=9). A participant with two or more AEs within the same level of summarisation (i.e., system, organ, class or preferred term) will be counted only once in that level. The number of AEs reported will also be presented. Adverse events will also be summarised by severity as well as relationship to study treatment. A by-participant AE data listing, including verbatim term, preferred term, system organ class, severity, and relationship to study treatment, will be provided. Separate listings will be generated for SAEs and AEs leading to study/treatment discontinuation.

All haematology, blood chemistry and urinalysis (continuous variables) parameters will be summarised using descriptive statistics for all study visits assessed, including change from baseline (last pre-surgery value) for all post-surgery assessments. All laboratory data will be included in the data listings and all test values outside the normal range will be flagged.

All vital sign parameters will be summarised using descriptive statistics by study visit, including change from baseline (last pre-surgery) for all post-surgery assessments.

Individual vital sign assessments will be listed for each participant. Findings of physical examinations will be listed for each participant and summarised descriptively by using count and percentage by study visit.

### ANALYSIS OF SECONDARY ENDPOINTS

The efficacy endpoints are the secondary endpoints of this study, which include complete ocular examination.

ETDRS best-corrected visual acuity (BCVA) will be scored with reference to the Early Treatment Diabetic Retinopathy Study ETDRS letters). ETDRS will be treated as continuous data, and descriptive statistics (mean, SD, median, minimum and maximum) will be summarised for ETDRS observed value and change from baseline at each post-surgery visit. Exploratory analysis of ETDRS change over time will be assessed by using a mixed model. The correlations between repeated measures of the same participant will be accounted for by the mixed model. The least squares mean of ETDRS change from baseline and its 95% confidence interval at each visit will be estimated. Similar analyses will be conducted for intraocular pressures. Categorical efficacy endpoints such as slit lamp biomicroscopy exam findings, dilated ophthalmoscopy exam findings, color fundus photography and OCT finding will be summarised descriptively by frequency count and percentage (proportion) where appropriate.

### INTERIM ANALYSIS

There is no formal interim analysis planned for this study.

### SAMPLE SIZE

The study is planned to enrol up to 12 participants with geographic atrophy secondary to age-related macular degeneration with visual acuity (20/32 to 20/2000, Snellen's equivalent), following the 3+3 method.

The sample size chosen for this study was selected without formal statistical justification, but the numbers chosen are considered adequate for assessing the study objectives. The sample size was determined on the basis of practical and logistical considerations and not based on statistical power with regard to hypothesis testing or precision with regard to parameter estimation.

This phase 1 trial was designed to identify any important limiting toxicities and to determine if this dose is suitable for phase 2 trial. This was also designed to minimize the likelihood that a minimum number of subjects will be exposed to the investigational drug.

This is an open label study, and no randomization is conducted in this study.

### MISSING DATA

Missing data will generally not be imputed for safety or efficacy data.

### DATA COLLECTION, RETENTION AND MONITORING

### DATA COLLECTION INSTRUMENTS

The investigator will prepare and maintain adequate and accurate source documents designed to record all observations and other pertinent data for each participant treated with the study drug.

Study personnel at each site will enter data from source documents corresponding to a participant's visit into the protocol-specific electronic Case Report Form (eCRF) when the information corresponding to that visit is available. Participants will not be identified by name in the study database or on any study documents to be collected by the Sponsor (or designee), but will be identified by a site number, participant number and initials.

If a correction is required for an eCRF, the time and date stamps track the person entering or updating eCRF data and creates an electronic audit trail. The Investigator is responsible for all information collected on participants enrolled in this study. All data collected during the course of this study must be reviewed and verified for completeness and accuracy by the Investigator. A copy of the CRF will remain at the Investigator's site at the completion of the study.

### DATA MANAGEMENT PROCEDURES

The data will be entered into a validated database. The Data Management group will be responsible for data processing, in accordance with procedural documentation. Database lock will occur once quality assurance procedures have been completed.

All procedures for the handling and analysis of data will be conducted using good computing practices meeting FDA guidelines for the handling and analysis of data for clinical trials.

### DATA QUALITY CONTROL AND REPORTING

After data have been entered into the study database, a system of computerised data validation checks will be implemented and applied to the database on a regular basis.

Queries are entered, tracked, and resolved through the EDC system directly. The study database will be updated in accordance with the resolved queries. All changes to the study database will be documented.

### ARCHIVAL DATA

The database is safeguarded against unauthorised access by established security procedures; appropriate backup copies of the database and related software files will be maintained. Databases are backed up by the database administrator in conjunction with any updates or changes to the database.

At critical junctures of the protocol (e.g., production of interim reports and final reports), data for analysis is locked and cleaned per established procedures.

### AVAILABILITY AND RETENTION OF INVESTIGATIONAL RECORDS

The Investigator must make study data accessible to the monitor, other authorized representatives of the Sponsor (or designee), IRB/IEC, and Regulatory Agency (e.g., FDA, TGA) inspectors upon request. A file for each participant must be maintained that includes the signed informed Consent, HIPAA Authorization and Assent Form and copies of all source documentation related to that participant. The Investigator must ensure the reliability and availability of source documents from which the information on the eCRF was derived.

All study documents (patient files, signed informed consent forms, copies of eCRFs,

Study File Notebook, etc.) must be kept secured for a period of fifteen years following the completion of the study.

### MONITORING

Monitoring visits will be conducted by representatives of the Sponsor according to the U.S. CFR Title 21 Parts 50, 56, and 312 and ICH Guidelines for GCP (E6). By signing this protocol, the investigator grants permission to the Sponsor (or designee), and appropriate regulatory authorities to conduct on-site monitoring and/or auditing of all appropriate study documentation.

### DATA SAFETY MONITORING BOARD

An independent DSMB will be convened. The mission of the DSMB will be to ensure the ethical conduct of the trial and to protect the safety interests of patients in this study.

The DSMB will be responsible for reviewing the cumulative safety results from the study. The DSMB will meet prior to the commencement of the study, and will review all available safety/tolerability data (e.g., adverse events, serious adverse events, clinical laboratory assessments, blood pressure, haematology, urology) at Day 0 and 1 month after IVT injection of FA of the initial participant in Part 1 and 3 (prior to Part 2 & 4 of the study), and convene as required throughout the study period to review data and potential safety risks. The criteria for evaluating study continuation will relate to study safety, including the incidence and severity of ocular and/or systemic side effects not limited to but including; change in IOP of >10mmHg, a loss of 15 letters or more in BCVA, presence or intraocular inflammation, presence or absence of ocular pain, change in BP of 30mmHg (systolic or diastolic), incidence of hospitalisation or systemic illness, and any other ocular or systemic adverse events reported. Any changes will be referenced to baseline measurements. The DSMB will then meet at the conclusion of the study and after the final statistical analysis in order to review all data.

DSMB will consist an ophthalmologist, and a biostatistician, both independent of the study team.

### PARTICIPANT CONFIDENTIALITY

In order to maintain participant confidentiality, only a site number, participant number and participant initials will identify all study participants on eCRFs and other documentation submitted to the Sponsor. Additional participant confidentiality issues (if applicable) are covered in the Clinical Study Agreement.

### ADMINISTRATIVE, ETHICAL, REGULATORY CONSIDERATIONS

The study will be conducted according to the Declaration of Helsinki, Protection of Human Volunteers (21 CFR 50), Institutional Review Boards (21 CFR 56), and Obligations of Clinical Investigators (21 CFR 312).

To maintain confidentiality, all laboratory specimens, evaluation forms, reports and other records will be identified by a coded number and initials only. All study records will be kept in a locked file cabinet and code sheets linking a patient's name to a patient identification number will be stored separately in another locked file cabinet. Clinical information will not be released without written permission of the participant, except as necessary for monitoring by the TGA. The Investigator must also comply with all applicable privacy regulations (e.g., The Health Records and Information Privacy Act 2002).

### PROTOCOL AMENDMENTS

Any amendment to the protocol will be written by the Sponsor. Protocol amendments cannot be implemented without prior written IRB/IEC approval except as necessary to eliminate immediate safety hazards to patients. A protocol amendment intended to eliminate an apparent immediate hazard to patients may be implemented immediately, provided the IRBs are notified within five working days.

### INSTITUTIONAL REVIEW BOARDS AND INDEPENDENT ETHICS COMMITTEE

The protocol and consent form will be reviewed and approved by the IRB/IEC of each participating centre prior to study initiation. Serious adverse events regardless of causality will be reported to the IRB/IEC in accordance with the standard operating procedures and policies of the IRB/IEC, and the Investigator will keep the IRB/IEC informed as to the progress of the study. The Investigator will obtain assurance of IRB/IEC compliance with regulations.

Any documents that the IRB/IEC may need to fulfil its responsibilities (such as protocol, protocol amendments, Investigator's Brochure, consent forms, information concerning patient recruitment, payment or compensation procedures, or other pertinent information) will be submitted to the IRB/IEC. The IRB/IECs written unconditional approval of the study protocol and the informed consent form will be in the possession of the Investigator before the study is initiated. The IRB/IECs unconditional approval statement will be transmitted by the Investigator to the Sponsor or designee prior to the shipment of study supplies to the site. This approval must refer to the study by exact protocol title and number and should identify the documents reviewed and the date of review.

Protocol and/or informed consent modifications or changes may not be initiated without prior written IRB/IEC approval except when necessary to eliminate immediate hazards to the patients or when the change(s) involves only logistical or administrative aspects of the study. Such modifications will be submitted to the IRB/IEC and written verification that the modification was submitted and subsequently approved should be obtained.

The IRB/IEC must be informed of revisions to other documents originally submitted for review; serious and/or unexpected adverse events occurring during the study in accordance with the standard operating procedures and policies of the IRB; new information that may affect adversely the safety of the patients of the conduct of the study; an annual update and/or request for re-approval; and when the study has been completed.

### INFORMED CONSENT FORM (ICF)

Informed consent will be obtained in accordance with the Declaration of Helsinki, ICH GCP, US Code of Federal Regulations for Protection of Human Subjects (21 CFR 50.25 [a,b], CFR 50.27, and CFR Part 56, Subpart A), the Health Insurance Portability and Accountability Act (HIPAA, if applicable), and local regulations.

Figures 2 to 6 show the results of injection of fludrocortisone into one subject according to the Phase 1b study. This data is for a single subject with bi-lateral GA, who was treated in the left eye with 1 mg intravitreal FA. There is no evidence of toxicity at day 28, no intra ocular pressure (IOP) and improved visual acuity (VA). The study also included urine pregnancy test (negative); vital signs examination; external eye exam (all normal); slit lamp biomicroscopy (all normal, apart from a previous scar on the anterior chamber); IOP measurement; dilated fundus examination (all normal apart from GA on left eye (OS) into which the injection was made and also in the right eye (OD); vitreous haze gracing (absent); spectral domain optical coherence tomography (SD OCT); and a review of body systems (all normal; apart from unrelated issues).

In this specification, the terms "comprises", "comprising" or similar terms are intended to mean a non-exclusive inclusion, such that an apparatus that comprises a list of elements does not include those elements solely, but may well include other elements not listed.

Throughout the specification the aim has been to describe the invention without limiting the invention to any one embodiment or specific collection of features. Persons skilled in the relevant art may realize variations from the specific embodiments that will nonetheless fall within the scope of the invention.

### Tables

**Table 1: Mineralocorticoid Receptor and Glucocorticoid Receptor activity of some corticosterones**

| **Compound** | **GR potency** | **MR potency** | **Duration of action** (t_{1/2} in hours) |
|---|---|---|---|
| Hydrocortisone (cortisol) | 1 | 1 | 8 |
| Cortisone | 0.8 | 0.8 | oral 8; i.m. 18+ |
| Prednisone | 3.5-5 | 0.8 | 16-36 |
| Prednisolone | 4 | 0.8 | 16-36 |
| Methylprednisolone | 5-7.5 | 0.5 | 18-40 |
| Dexamethasone | 25-80 | 0 | 36-54 |
| Betamethasone | 25-30 | 0 | 36-54 |
| Triamcinolone | 5 | 0 | 12-36 |
| Beclometasone | 8 puffs 4 times a day; equals 14 mg oral prednisone once/day | - | - |
| Fludrocortisone acetate | 15 | 200 | 24 |
| Deoxycorticosterone acetate (DOCA) | 0 | 20 | - |
| Aldosterone | 0.3 | 200-1000 | - |

| | | | |
|---|---|---|---|
| Key: MR = mineralocorticoid receptor; GR = glucocorticoid receptor; i.m. intramuscular | | | |

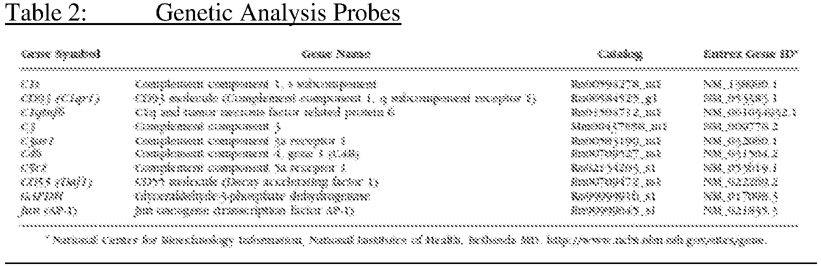

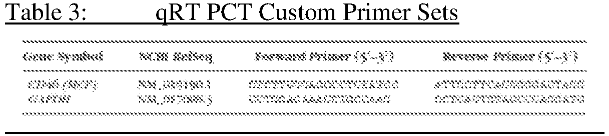

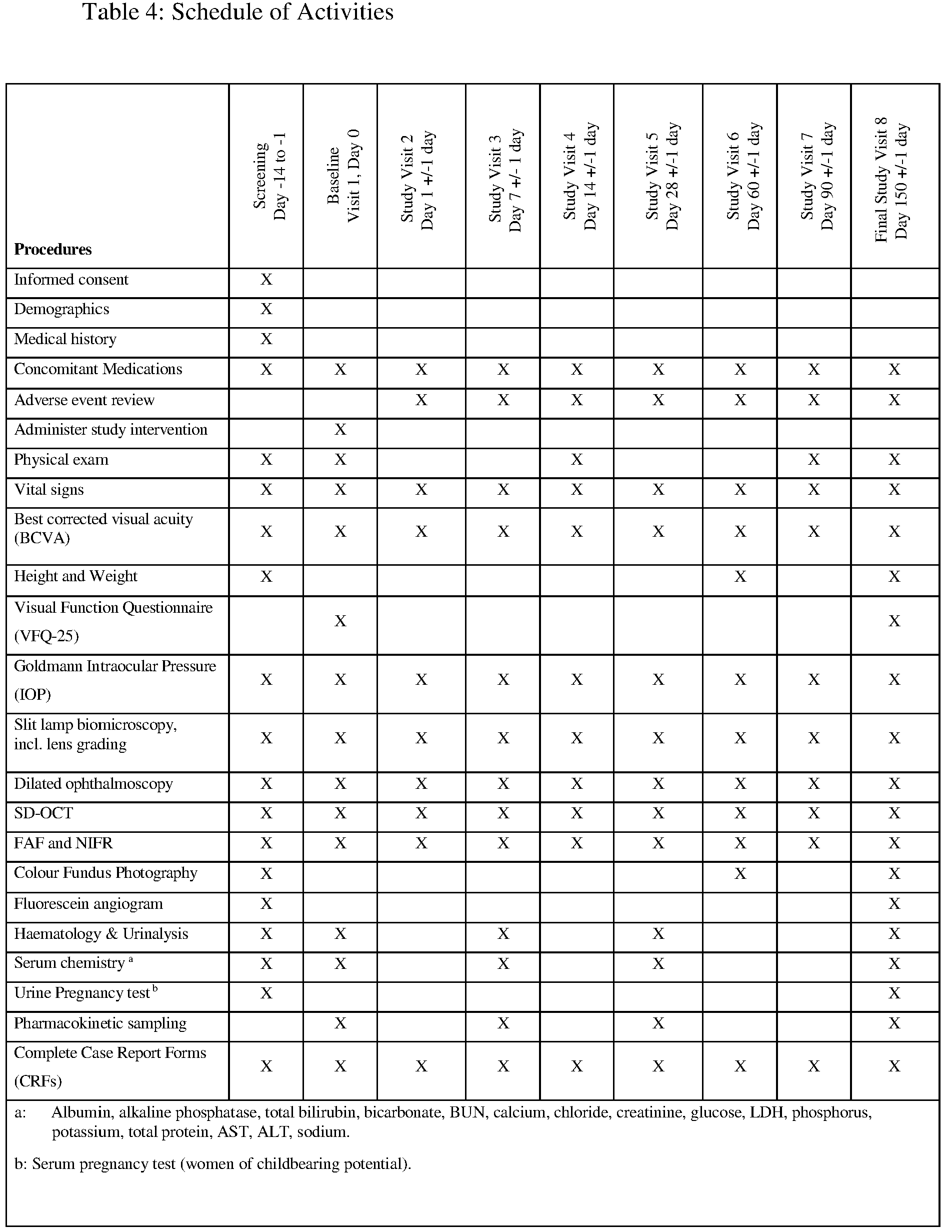

**Table 5:**

| | Treatment Arm | Dose Escalation |
|---|---|---|
| **Part 1** | Fludrocortisone acetate | If no dose limiting toxicity is observed in 3 patients at this given dose level, the dose will be escalated to the following level: |
| | 1 mg/0.1mL at Day 0 | |
| **Part 2** | Fludrocortisone acetate | |
| | 2 mg/0.1 mL | |

**Table 6:**

| **As say** | **Number of Time Points** | **Approximate Volume per Time Point (mL)** | **Approximate Sample Volume Over Course of Study (mL)** |
|---|---|---|---|
| Pharmacokinetics | 4/9 | 4 | 36 |
| Haematology | 5/9 | 4 | 36 |
| Chemistry (Incl. HCG/LH/FSH) | 5/9 | 8.5 | 76.5 |

### REFERENCES

Aruna Gorusupudi, Kelly Nelson, and Paul S Bernstein, The Age-Related Eye Disease 2 Study: Micronutrients in the Treatment of Macular Degeneration. American Society for Nutrition. Adv Nutr. 2017;8:40-53; doi:10.3945/an.116.013177.
Rishi P. Singh, Advances in the Treatment of Dry Age-Related Macular Degeneration: Preventing and treating geographic atrophy: https://consultqd.clevelandclinic.org/2017/01/advances-treatment-dry-age-related-macular-degeneration/
Riccardo Natoli, Nilisha Fernando, Michele Madigan, Joshua A. Chu-Tan, Krisztina Valter, Jan Provis and Matt Rutar, Microglia-derived IL-1β promotes chemokine expression by Müller cells and RPE in focal retinal degeneration. Molecular Neurodegeneration (2017) 12:31 DOI 10.1186/s13024-017-0175-y
(Natoli et al. b) Riccardo Natoli, Nilisha Fernando, Haihan Jiao, Tanja Racic, Michele Madigan, Nigel L. Barnett, Joshua A. Chu-Tan, Krisztina Valter, Jan Provis, and Matt Rutar, Retinal Macrophages Synthesize C3 and Activate Complement in AMD and in Models of Focal Retinal Degeneration. IOVS, June 2017, 58:7, pp 2977-2990
http://iovs.arvojournals.org/pdfaccess.ashx?url=/data/journals/iovs/936282/
Matt Rutar, Riccardo Natoli, Peter Kozulin, Krisztina Valter, Paul Gatenby, and Jan M. Provis, Analysis of Complement Expression in Light-Induced Retinal Degeneration: Synthesis and Deposition of C3 by Microglia/Macrophages Is Associated with Focal Photoreceptor Degeneration. Investigative Ophthalmology & Visual Science, July 2011, Vol. 52, No. 8.
Rozen S, Skaletsky H. Primer3 on the WWW for general users and for biologist programmers. Methods Mol Biol. 2000;132:365-386.

## Claims

1. A pharmaceutical composition comprising a therapeutically effective amount of fludrocortisone, fludrocortisone acetate or fludrocortisone acetonide for use in the treatment of geographic atrophy.

2. The composition for the use of claim 1 wherein the fludrocortisone, fludrocortisone acetate or fludrocortisone acetonide is capable of modulating the activity of both a mineralocorticoid receptor and a glucocorticoid receptor.

3. The composition for the use of any one of the above claims further comprising triamcinolone or triamcinolone acetonide.

4. The composition for the use of any one of claims 1 to 3 wherein the composition further comprises one or more mineralocorticoid.

5. The composition for the use of any one of the above claims wherein the pharmaceutical composition is injected into the eye.

6. The composition for the use of any one of the above claims wherein the at least one anti-inflammatory is provided in a unit-dose formulation.

7. The composition for the use of any one of the above claims wherein the composition is preservative free.

8. The composition for the use of any one of the above claims wherein the fludrocortisone; fludrocortisone acetate; or fludrocortisone acetonide comprises a sustained release fludrocortisone; fludrocortisone acetate; or fludrocortisone acetonide or a sustain release composition.

9. The composition for the use of any one of the above claims wherein the fludrocortisone; fludrocortisone acetate; or fludrocortisone acetonide or composition is sterilized.

10. The composition for the use of any one of the above claims further comprising at least one additional agent or administering at least one additional agent.

11. The composition for the use of claim 10 wherein the additional agent comprises an anti-VEGF (anti-Vascular Endothelial Growth Factor).

12. The composition for the use of claim 11 wherein the anti-VEGF comprises one or more of ranibizumab (brand name Lucentis^{®}); aflibercept (brand name Eylea^{®}); bevacizumab (brand name Avastin^{®}) and OPT-302.

13. The composition for the use of any one of the above claims wherein treatment comprises prophylactic treatment.

14. The composition for the use of any one of claims 3 to 13 wherein the triamcinolone and/or triamcinolone acetonide is comprised in a concentration of 3.0 to 5.0 mg/ml.

## Patentansprüche

1. Eine Pharmazeutische Zusammensetzung, umfassend eine therapeutisch wirksame Menge Fludrocortison, Fludrocortisonacetat oder Fludrocortisonacetonid zur Verwendung bei der Behandlung von geographischer Atrophie.

2. Die Zusammensetzung zur Verwendung nach Anspruch 1, wobei das Fludrocortison, Fludrocortisonacetat oder Fludrocortisonacetonid in der Lage ist, die Aktivität sowohl eines Mineralocorticoidrezeptors als auch eines Glucocorticoidrezeptors zu modulieren.

3. Die Zusammensetzung zur Verwendung nach einem der obigen Ansprüche, zudem umfassend Triamcinolon oder Triamcinolonacetonid.

4. Die Zusammensetzung zur Verwendung nach einem der Ansprüche 1 bis 3, wobei die Zusammensetzung weiterhin ein oder mehrere Mineralocorticoide enthält.

5. Die Zusammensetzung zur Verwendung nach einem der obigen Ansprüche, wobei die pharmazeutische Zusammensetzung in das Auge injiziert wird.

6. Die Zusammensetzung zur Verwendung nach einem der obigen Ansprüche, wobei der mindestens eine Entzündungshemmer in einer Einheitsdosisformulierung bereitgestellt wird.

7. Die Zusammensetzung zur Verwendung nach einem der obigen Ansprüche, wobei die Zusammensetzung frei von Konservierungsmitteln ist.

8. Die Zusammensetzung zur Verwendung nach einem der obigen Ansprüche, wobei das Fludrocortison; Fludrocortisonacetat; oder Fludrocortisonacetonid verzögerte Freisetzung von Fludrocortison; Fludrocortisonacetat; oder Fludrocortisonacetonid oder eine Zusammensetzung mit verzögerter Freisetzung umfasst.

9. Die Zusammensetzung zur Verwendung nach einem der obigen Ansprüche, wobei das Fludrocortison; Fludrocortisonacetat; oder Fludrocortisonacetonid oder die Zusammensetzung sterilisiert ist.

10. Die Zusammensetzung zur Verwendung nach einem der obigen Ansprüche, zudem umfassend mindestens ein zusätzliches Mittel oder die Verabreichung mindestens eines zusätzlichen Mittels.

11. Die Zusammensetzung zur Verwendung nach Anspruch 10, wobei das zusätzliche Mittel einen Anti-VEGF (Anti-Vascular Endothelial Growth Factor) umfasst.

12. Die Zusammensetzung für die Verwendung nach Anspruch 11, wobei der Anti-VEGF eines oder mehrere von Ranibizumab (Markenname Lucentis^{®}); Aflibercept (Markenname Eylea^{®}); Bevacizumab (Markenname Avastin^{®}) und OPT-302 umfasst.

13. Die Zusammensetzung zur Verwendung nach einem der obigen Ansprüche, wobei die Behandlung eine prophylaktische Behandlung umfasst.

14. Die Zusammensetzung zur Verwendung nach einem der Ansprüche 3 bis 13, wobei das Triamcinolon und/oder Triamcinolonacetonid in einer Konzentration von 3,0 bis 5,0 mg/ml umfasst ist.

## Revendications

1. Composition pharmaceutique comprenant une quantité thérapeutiquement efficace de fludrocortisone, d'acétate de fludrocortisone ou d'acétonide de fludrocortisone pour utilisation dans le traitement de l'atrophie géographique.

2. Composition pour utilisation selon la revendication 1, dans laquelle la fludrocortisone, l'acétate de fludrocortisone ou l'acétonide de fludrocortisone est capable de moduler l'activité à la fois d'un récepteur de minéralocorticoïde et d'un récepteur de glucocorticoïde.

3. Composition pour utilisation selon l'une quelconque des revendications précédentes, comprenant en outre de la triamcinolone ou de l'acétonide de triamcinolone.

4. Composition pour utilisation selon l'une quelconque des revendications 1 à 3, dans laquelle la composition comprend en outre un ou plusieurs minéralocorticoïdes.

5. Composition pour utilisation selon l'une quelconque des revendications précédentes, dans laquelle la composition pharmaceutique est injectée dans l'œil.

6. Composition pour utilisation selon l'une quelconque des revendications précédentes, dans laquelle le au moins un anti-inflammatoire est fourni dans une formulation à dose unitaire.

7. Composition pour utilisation selon l'une quelconque des revendications précédentes, dans laquelle la composition est exempte d'agent de conservation.

8. Composition pour utilisation selon l'une quelconque des revendications précédentes, dans laquelle la fludrocortisone ; l'acétate de fludrocortisone ; ou l'acétonide de fludrocortisone comprend une fludrocortisone à libération prolongée ; de l'acétate de fludrocortisone ; ou de l'acétonide de fludrocortisone ou une composition à libération prolongée.

9. Composition pour utilisation selon l'une quelconque des revendications précédentes, dans laquelle la fludrocortisone ; l'acétate de fludrocortisone ; ou l'acétonide ou la composition de fludrocortisone est stérilisé(e).

10. Composition pour utilisation selon l'une quelconque des revendications précédentes, comprenant en outre au moins un agent supplémentaire ou l'administration d'au moins un agent supplémentaire.

11. Composition pour utilisation selon la revendication 10, dans laquelle l'agent supplémentaire comprend un anti-VEGF (anti-facteur de croissance de l'endothélium vasculaire).

12. Composition pour utilisation selon la revendication 11, dans laquelle l'anti-VEGF comprend un ou plusieurs parmi le ranibizumab (nom de marque Lucentis(t) ; l'aflibercept (nom de marque Eylea^{®}) ; le bevacizumab (nom de marque Avastin^{®}) et OPT-302.

13. Composition pour utilisation selon l'une quelconque des revendications précédentes, dans laquelle le traitement comprend un traitement prophylactique.

14. Composition pour utilisation selon l'une quelconque des revendications 3 à 13, dans laquelle la triamcinolone et/ou l'acétonide de triamcinolone est compris à une concentration de 3,0 à 5,0 mg/ml.
